# EUROPEAN PATENT APPLICATION

(11) **EP 4 654 208 A2**
(43) Date of publication of application: **26.11.2025**
(21) Application number: 25206135.3
(22) Date of filing: 11.09.2020
(51) Int. Cl.: G16B 50/50

(54) **METHOD FOR THE COMPRESSION OF GENOME SEQUENCE DATA**

(30) Priority: 11.09.2019 US 201916567211
(62) Divisional of application: 20780856.9
(71) Applicant: ILLUMINA, INC., San Diego, CA 92122 (US)
(72) Inventor: RIZK, Guillaume Alexandre Pascal, San Diego, 92122 (US)
(74) Representative: Lowden, Samuel Robert James

(57) **Abstract**

A reference-based method is provided for the compression of genome sequence data produced by a sequencing machine. Reads of nucleotides or bases, that have been previously aligned to a reference sequence, are determined to be perfectly mapped, imperfectly mapped or unmapped with the reference sequence; and then encoded according to said determination. The method comprises comparing, for each imperfectly mapped read, the number of mismatches between said sequence and the reference sequence with a reference threshold value, and encoding the imperfectly mapped reads according to distinct encoding processes, depending on the result of said comparison.

## Description

### Technical Field

The field relates generally to methods of representation of genome sequencing data produced by a sequencing machine, and more particularly to the computer-implemented methods for the compression of such genome sequencing data. This disclosure provides a reference-based compression method which allows fast compression and decompression while causing no loss of information, and which has a high compression ratio.

### Background

Next generation sequencing machines now produce huge amounts of sequencing data at an affordable price. Recent systems produce in a single run of 36h more than 6 billion 150-nucleotide long sequences, enough for the sequencing of 20 whole human genomes. This opens many new perspectives for the diagnostic of genetic diseases and for the development of personalized medicine, aiming to adapt treatment based on people genomic specificities.

However, this also comes with new challenges, in particular the cost related to the storage of huge amounts of data. The most used file format for raw (unaligned) sequence data is the FASTQ format, holding sequence data (string of A, C, T, G nucleotides, also called read), quality values (probabilities that the sequencing platform made a sequencing error for each nucleotide) and sequence names. This is a plain ASCII text file, usually compressed with the general purpose text compression scheme LZ (Lempel-Ziv scheme, implemented in the gzip software). However, the use of such compression methods comes with several issues:
- low compression ratio because the redundancy of the data is not fully used
- slow compression and decompression

There also exists compression methods specialized in FASTQ encoding, divided in reference or non reference-based methods. However, none of them are fully satisfying, since a) the reference-based methods have good compression ratios but are slow, b) the non reference-based methods are faster but have lower compression ratios. An example of such a non reference-based method is provided by the software SPRING, which is a reference-free compressor for FASTQ files (worldwide web address: github.com/shubhamchandak94/SPRING). However, the compression method provided by the software SPRING has a low compression ratio.

Among the reference-based compression methods, some methods that use sequence alignments and are aimed to be faster with good compression ratios have been proposed. However, such methods suffer from several problems, notably a major issue is that they are not completely lossless. Such a known reference-based compression method is for example described in the patent document WO 2018/068829 A1. In the described method, after having been aligned to one or more reference sequences, the sequences of nucleotides are classified according to matching accuracy degrees (thereby creating classes of aligned reads), and are then coded as a multiplicity of layers of syntax elements, using different source models and entropy coders for each layer in which the data is partitioned. The classes of data are thus encoded separately and are structured in different layers of syntax elements, each layer comprising descriptors which univocally represent the classified and aligned reads of said layer. The method is intended to obtain distinct information sources with reduced information entropy, thereby allowing an increase in compression performance as well as a selective access to specific classes of compressed data. However, such a compression method reorders the reads in an order that is different from that obtained at the end of the read alignment step (i.e. the reads are reordered according to their classes). Some information is then lost in the compression process, notably the initial sequence ordering. Hence the reproducibility of some analysis results can be affected, because some downstream analysis software can be dependent on the order of the reads. Besides, decompressing the data in an order that is different from the initial order of the reads makes it much more difficult to check that the uncompressed file is identical to the initial file. Furthermore, such a compression method is relatively slow, especially when compared to the non-reference-based compression methods of the state of the art.

### Summary

The present disclosure solves the problem of existing prior art solutions by providing systems, methods, computer programs, and hardware circuitry for the compression of genome sequence data. In one aspect, computer-implemented methods for the compression of genome sequence data produced by a sequencing machine, said genome sequence data including reads of sequences of nucleotides or bases that have been aligned to a reference sequence, thereby creating aligned reads, said aligned reads being stored as a list of reads in an initial file, includes:
- for each aligned read, determining whether said read is perfectly or imperfectly mapped with said reference sequence or whether said read is unmapped with said reference sequence,
- encoding the reads according to said determination, wherein the reads that are determined to be perfectly mapped are encoded according to a first encoding process and the reads that are determined to be unmapped are encoded according to a second encoding process,
- wherein the determining step comprises comparing, for each imperfectly mapped read, the number of mismatches between said read and said reference sequence with a threshold value,
- wherein, in the encoding step, the reads that are determined to be imperfectly mapped are encoded according to the second encoding process or to a third encoding process, the imperfectly mapped reads being encoded according to the second encoding process when said number of mismatches is greater than the threshold value, the imperfectly mapped reads being encoded according to the third encoding process when said number of mismatches is lower than the threshold value,
- wherein, in said second encoding process, each nucleotide or base of the read is individually encoded,
- wherein said first and third encoding processes comprise distinct sets of descriptors, each set of descriptors univocally representing the reads associated to the corresponding encoding process, each of said first and third encoding processes being a reduced information source entropy encoding process.

The present disclosure overcomes the disadvantages of prior compression methods by allowing fast compression and decompression while causing no loss of information, and providing a high compression ratio. More particularly, the present disclosure focuses on encoding the most frequent cases in the most compact way, even if this means adopting degraded encoding modes for the rare least frequent cases. This leads to a huge increase in compression performance. Moreover, due to the genomic information representation format that is used by the present disclosure, the compression performed by the methods described herein are faster. Last but not least, the present disclosure keeps the initial order of the reads as such and does not reorder the reads according to their classes. Consequently, no information is lost during the process, which enables an easier downstream analysis as well as efficient conformity checks after the decompression step.

These and other features and advantages of the present disclosure will become more apparent from the accompanying drawings and the following detailed description. In addition, though thresholds may be referred to herein as being exceeded or not exceeded, it is understood that such thresholds can be conceptually employed so that it is determined whether such threshold is satisfied, met, or otherwise detected, regardless of whether the numbers or values used to implement those threshold evaluations are described using positive or negative values.

In accordance with one innovative aspect of the present disclosure, a method for compressing genomic sequence data is disclosed. In one aspect, the method can include performance of one or more operations via execution of software instructions by one or more computers, where the operations include that include obtaining, by the one or more computers, a read record, determining, by the one or more computers, whether the read record corresponds to a read that is perfectly mapped to a reference sequence or imperfectly mapped to the reference sequence, based on determining, by the one or more computers, that the read record corresponds to a read that is imperfectly mapped to the reference sequence, determining, by the one or more computers, whether a number of mismatches of the imperfectly mapped read satisfies a predetermined threshold number of mismatches, and based on determining that the number of mismatches satisfies the predetermined threshold number of mismatches, encoding, by the one or more computers, each mismatch of the imperfectly mapped read into a record having a size of 1 byte.

Other aspects include corresponding systems, apparatus, and computer programs to perform the actions of methods as disclosed herein as defined by instructions encoded on computer readable storage devices.

These and other versions may optionally include one or more of the following features. For instance, in some implementations, determining, by the one or more computers, whether a number of mismatches of the imperfectly mapped read satisfies a predetermined threshold number of mismatches can include determining, by the one or more computers, whether the number of mismatches of the imperfectly mapped read is greater than the predetermined threshold number of mismatches.

In some implementations, each read record can include data indicating an absolute starting position of an aligned read with respect to the reference sequence, data indicating a length of the read, data indicating whether the read is perfectly mapped or imperfectly mapped, data indicating a number of mismatches identified in the read, and data indicating a relative position of each of said possible mismatches in the read.

In some implementations, encoding each mismatch of the imperfectly mapped read into a record having a size of 1 byte comprises for each particular mismatch: encoding, by the one or more computers, a first two bits of the byte to include data representing an alternate nucleotide or base present in the read instead of a corresponding reference nucleotide or base in the reference sequence, and encoding, by one or more computers, six remaining bits of the byte to include data representing a position of the mismatch in the reference sequence, said position being computed as an offset from a previous mismatch of the read.

In some implementations, the method can further include determining, by one or more computers, whether the offset is greater than a maximum encodable value, and based on determining that the offset is greater than the maximum encoded value, inserting, by one or more computers, at least one fake mismatch between the particular mismatch and the previous mismatch.

In some implementations, the method can further include based on determining that the number of mismatches does not satisfy the predetermined threshold number of mismatches, encoding, by one or more computers, a list of positions of the reference sequence corresponding to a position of each of the mismatches to the reference sequence using a reduced information entropy encoding process.

In some implementations, the method further can further include based on determining that the read record corresponds to a read that is perfectly mapped to the reference sequence, encoding, by one or more computers, at least a portion of the read record using reduced information entropy encoding.

In some implementations, the one or more computers can include one or more hardware processors.

In some implementations, the one or more hardware processors can include one or more field programmable gate arrays (FPGAs).

In some implementations, the method for compressing genomic sequence data can be performed by one or more hardware processors. In such implementations, the hardware processors can include hardware processing circuitry that is configured to perform one or more operations. In one aspect, the operations can include obtaining, by the hardware processing circuitry, a read record, determining, by the hardware processing circuitry, whether the read record corresponds to a read that is perfectly mapped to a reference sequence or imperfectly mapped to the reference sequence, based on determining, by the hardware processing circuitry, that the read record corresponds to a read that is imperfectly mapped to the reference sequence, determining, by the one or more computers, whether a number of mismatches of the imperfectly mapped read satisfies a predetermined threshold number of mismatches, and based on determining that the number of mismatches satisfies the predetermined threshold number of mismatches, encoding, by the hardware processing circuitry, each mismatch of the imperfectly mapped read into a record having a size of 1 byte.

In some implementations, each read record can include: data indicating an absolute starting position of the aligned read with respect to the reference sequence, data indicating a length of the read, data indicating whether the read is perfectly mapped or imperfectly mapped, data indicating a number of mismatches identified in the read, and data indicating a relative position of said possible mismatches in the read.

In some implementations, determining, by the hardware processing circuitry, whether a number of mismatches of the imperfectly mapped read satisfies a predetermined threshold number of mismatches can include determining, by the hardware processing circuitry, whether the number of mismatches of the imperfectly mapped read is greater than the predetermined threshold number of mismatches.

In some implementations, encoding each mismatch of the imperfectly mapped read into a record having a size of 1 byte can include for each particular mismatch encoding, by the hardware processing circuitry, a first two bits of the byte to include data representing an alternate nucleotide or base present in the read instead of a corresponding reference nucleotide or base in the reference sequence, and encoding, by the hardware processing circuitry, a six remaining bits of the byte to include data representing a position of the mismatch in the reference sequence, said position being computed as an offset from a previous mismatch of the read.

In some implementations, the hardware processor circuitry is further configured to perform operations that include determining, by the hardware processing circuitry, whether the offset is greater than a maximum encodable value, and based on determining that the offset is greater than the maximum encoded value, inserting, by the hardware processing circuitry, at least one fake mismatch between the particular mismatch and the previous mismatch.

In some implementations, the hardware processor circuity is further configured to perform operations that include based on determining that the number of mismatches does not satisfy the predetermined threshold number of mismatches, encoding, by the hardware processing circuitry, a list of positions of the reference sequence corresponding to a position of each of the mismatches to the reference sequence using a reduced information entropy encoding process.

In some implementations, the hardware processor circuitry is further configured to perform operations that include based on determining that the read record corresponds to a read that is perfectly mapped to the reference sequence, encoding, by the hardware processing circuitry, at least a portion of the read record using reduced information entropy encoding.

In some implementations, the hardware processing circuitry comprises one or more field programmable gate arrays (FPGAs).

According to another innovative aspsect of the present disclosure, a method for compressing genomic sequence data is disclosed. In one aspect, the method can include operations of accessing, by the one or more processors, a storage device storing a plurality of read records in manner that preserves a sequence ordering of the read records as the produced by a mapping and aligning module, for each particular read record of the plurality of read records: obtaining, by the one or more processors, the particular read record, determining, by the one or more processors, whether the particular read record corresponds to a read that is perfectly mapped to a reference sequence or imperfectly mapped to the reference sequence, based on determining, by the one or more processors, that the particular read record corresponds to a read that is imperfectly mapped to the reference sequence, determining, by the one or more processors, whether a number of mismatches of the imperfectly mapped read satisfies a predetermined threshold number of mismatches, based on determining that the number of mismatches satisfies the predetermined threshold number of mismatches, encoding, by the one or more processors, each mismatch of the imperfectly mapped read into a compressed record having a predetermined compressed record size, and storing, by the one or more processors, the compressed record in the storage device while maintaining the sequence ordering of the read records.

Other aspects include corresponding systems, apparatus, and computer programs to perform the actions of methods as disclosed herein as defined by instructions encoded on computer readable storage devices.

These and other versions may optionally include one or more of the following features. For instance, in some implementations, each read record of the plurality of read records can include data indicating an absolute starting position of the aligned read with respect to the reference sequence, data indicating a length of the read, data indicating whether the read is perfectly mapped or imperfectly mapped, data indicating a number of mismatches identified in the read, data indicating whether the read includes at least one undetermined base N, data indicating a number of undetermined bases N in the read, data indicating whether the read is mapped or unmapped, data indicating a position of the read record in a sequence of read records output by the mapping and aligning module, and data indicating a relative position of said possible mismatches in the read.

In some implementations, the predetermined compressed record size is one byte.

In some implementations, ncoding each mismatch of the imperfectly mapped read into a compressed record having a size of one byte can include for each particular mismatch encoding, by one or more processors, a first two bits of the byte to include data representing an alternate nucleotide or base present in the read instead of a corresponding reference nucleotide or base in the reference sequence, and encoding, by one or more processors, a six remaining bits of the byte to include data representing a position of the mismatch in the reference sequence, said position being computed as an offset from a previous mismatch of the read.

In some implementations, the method can further include determining, by one or more processors, whether the offset is greater than a maximum encodable value, and based on determining that the offset is greater than the maximum encoded value, inserting, by one or more processors, at least one fake mismatch between the particular mismatch and the previous mismatch.

In some implementations, the method can further include based on determining that the number of mismatches does not satisfy the predetermined threshold number of mismatches, encoding, by one or more processors, a list of positions of the reference sequence corresponding to a position of each of the mismatches to the reference sequence using a reduced information entropy encoding process.

In some implementations, the method can further include based on determining that the read record corresponds to a read that is perfectly mapped to the reference sequence, encoding, by the one or more processors, at least a portion of the read record using reduced information entropy encoding.

In some implementations, determining, by the one or more computers, whether a number of mismatches of the imperfectly mapped read satisfies a predetermined threshold number of mismatches can include determining, by the one or more processors, whether the number of mismatches of the imperfectly mapped read is greater than the reference threshold.

According to another innovative aspect of the present disclosure, a hardware processor is disclosed. In one aspect, the hardware processor can include hardware processing circuitry that is configured to perform one or more operations. In one aspect, the operations that the hardware processing circuity is configured to perform include accessing, by the hardware processing circuitry, a storage device storing a plurality of read records in manner that preserves a sequence ordering of the read records as the produced by a mapping and aligning module, for each particular read record of the plurality of read records: obtaining, by the hardware processing circuitry, the particular read record, determining, by the hardware processing circuitry, whether the particular read record corresponds to a read that is perfectly mapped to a reference sequence or imperfectly mapped to the reference sequence, based on determining, by the hardware processing circuitry, that the particular read record corresponds to a read that is imperfectly mapped to the reference sequence, determining, by the hardware processing circuitry, whether a number of mismatches of the imperfectly mapped read satisfies a predetermined threshold number of mismatches, based on determining that the number of mismatches satisfies the predetermined threshold number of mismatches, encoding, by the hardware processing circuitry, each mismatch of the imperfectly mapped read into a compressed record having a predetermined compressed record size, and storing, by the hardware processing circuitry, the compressed record in the storage device while maintaining the sequence ordering of the read records.

These and other versions may optionally include one or more of the following features. For instance, in some implementations, each read record of the plurality of read records that can be accessed by the hardware processing circuity can include data indicating an absolute starting position of the aligned read with respect to the reference sequence, data indicating a length of the read, data indicating whether the read is perfectly mapped or imperfectly mapped, data indicating a number of mismatches identified in the read, data indicating whether the read includes at least one undetermined base N, data indicating a number of undetermined bases N in the read, data indicating whether the read is mapped or unmapped, data indicating a position of the read record in a sequence of read records output by the mapping and aligning module, and data indicating a relative position of said possible mismatches in the read.

In some implementations, the predetermined compressed record size generated by the hardware processing circuitry can be one byte.

In some implementations, encoding each mismatch of the imperfectly mapped read into a compressed record having a size of one byte can include for each particular mismatch: encoding, by the hardware processing circuitry, a first two bits of the byte to include data representing an alternate nucleotide or base present in the read instead of a corresponding reference nucleotide or base in the reference sequence, and encoding, by the hardware processing circuitry, a six remaining bits of the byte to include data representing a position of the mismatch in the reference sequence, said position being computed as an offset from a previous mismatch of the read.

In some implementations, hardware processor can be further configured to include hardware processing circuity that is configured to perform operations that include determining, by the hardware processing circuitry, whether the offset is greater than a maximum encodable value, and based on determining that the offset is greater than the maximum encoded value, inserting, by the hardware processing circuitry, at least one fake mismatch between the particular mismatch and the previous mismatch.

In some implementations, hardware processor can be further configured to include hardware processing circuity that is configured to perform operations that include based on determining that the number of mismatches does not satisfy the predetermined threshold number of mismatches, encoding, by the hardware processing circuitry, a list of positions of the reference sequence corresponding to a position of each of the mismatches to the reference sequence using a reduced information entropy encoding process.

In some implementations, hardware processor can be further configured to include hardware processing circuity that is configured to perform operations that include based on determining that the read record corresponds to a read that is perfectly mapped to the reference sequence, encoding, by the hardware processing circuitry, at least a portion of the read record using reduced information entropy encoding.

In some implementations, determining, by the hardware processing circuitry, whether a number of mismatches of the imperfectly mapped read satisfies a predetermined threshold number of mismatches comprises:
determining, by the hardware processing circuitry, whether the number of mismatches of the imperfectly mapped read is greater than the predetermined threshold number of mismatches.

According to another innovative aspect of the present disclosure, a computer-implemented method for the compression of genome sequence data produced by a sequencing machine, said genome sequence data comprising reads of sequences of nucleotides or bases that have been aligned to a reference sequence, thereby creating aligned reads, said aligned reads being stored as a list of reads in an initial file. In one aspect, the method can include actions of for each aligned read, determining whether said read is perfectly or imperfectly mapped with said reference sequence or whether said read is unmapped with said reference sequence, encoding the reads according to said determination, wherein the reads that are determined to be perfectly mapped are encoded according to a first encoding process and the reads that are determined to be unmapped are encoded according to a second encoding process, wherein the determining step comprises comparing, for each imperfectly mapped read, the number of mismatches between said read and said reference sequence with a threshold value, wherein, in the encoding step, the reads that are determined to be imperfectly mapped are encoded according to the second encoding process or to a third encoding process, the imperfectly mapped reads being encoded according to the second encoding process when said number of mismatches is greater than the threshold value, and the imperfectly mapped reads being encoded according to the third encoding process when said number of mismatches is lower than the threshold value, wherein, in said second encoding process, each nucleotide or base of the read is individually encoded, wherein said first and third encoding processes comprise distinct sets of descriptors, each set of descriptors univocally representing the reads associated to a corresponding encoding process, each of said first and third encoding processes being a reduced information source entropy encoding process.

Other aspects include corresponding systems, apparatus, and computer programs to perform the actions of methods as disclosed herein as defined by instructions encoded on computer readable storage devices.

These and other versions may optionally include one or more of the following features. For instance, in some implementations, the determining step can include, when a read is determined to be imperfectly mapped with the reference sequence and has a number of mismatches lower than the threshold value, a further determination as to whether the read is globally or locally mapped with said reference sequence, and wherein the third encoding process comprises a first encoding subprocess and a second encoding subprocess, the reads that are determined to be globally mapped being encoded according to the first encoding subprocess, the reads that are determined to be locally mapped being encoded according to the second encoding subprocess, said first and second encoding subprocesses comprising distinct sets of descriptors, each set of descriptors univocally representing the reads associated to a corresponding encoding subprocess.

In some implementations, said descriptors of said first encoding subprocess can include an alignment start position in the reference sequence, a read length and a list of mismatches by substitutions of symbols, and wherein said descriptors of said second encoding subprocess comprise a local alignment start position in the reference sequence, a read length, a list of mismatches by substitutions of symbols, and a length of the clipped portions of the read that are not part of the alignment.

In some implementations, in the encoding step, the clipped portions of a read that is to be encoded according to the second encoding subprocess are concatenated, each nucleotide or base of said clipped portions being individually encoded.

In some implementations, in the encoding step, each mismatch of an imperfectly mapped read is encoded on 1 byte.

In some implementations, in the encoding step, each mismatch of an imperfectly mapped read is encoded as follows: two first bits of the byte are used to encode an alternate nucleotide or base present in the read instead of a corresponding reference nucleotide or base in the reference sequence, and six last bits of the byte are used to encode a position of the mismatch in the reference sequence, said position being computed as an offset from a previous mismatch of the read.

In some implementations, in the encoding step, if the offset computed between a given mismatch and the previous mismatch is greater than a maximum encodable value, then at least one fake mismatch is inserted between said two mismatches until every offset between each of said mismatches and said at least one fake mismatch is lower than said maximum encodable value, a fake mismatch being defined as a mismatch for which bits of the byte used to encode the mismatch or to encode a nucleotide or base that is equal to the corresponding reference nucleotide or base in the reference sequence.

In some implementations, an initial step of dividing the list of reads into blocks of reads, with each block beginning with a header containing information needed to decode the block, wherein said compression method is performed block by block.

In some implementations, blocks of reads have the same block size.

In some implementations, a final step of providing a compressed file comprising a list of encoded reads, said encoded reads being stored in the compressed file in the same order as that of the reads stored in the initial file.

In some implementations, said threshold value is equal to 31.

In some implementations, for each aligned read, a step of determining whether said read comprises at least one mismatch corresponding to a case in which the sequencing machine was unable to call any base or nucleotide.

In some implementations, for each read comprising at least one mismatch corresponding to a case in which the sequencing machine was unable to call any base or nucleotide, a step of determining the number of such mismatches and a step of comparing said number with a reference threshold value.

In some implementations, in the encoding step, if the number of such mismatches is greater than the reference threshold value, each nucleotide or base of a read that is to be encoded according to the second encoding process is individually encoded on 4 bits, and, if the number of such mismatches is lower than the reference threshold value, each nucleotide or base of a read that is to be encoded according to the second encoding process is individually encoded on 2 bits and the encoding step further comprises encoding a list of positions along the reference sequence, said positions corresponding to the positions of such mismatches in the reference sequence.

### Brief Description of the Drawings

Figure 1 is a flow diagram showing an example of a compression method described herein.
Figure 1A is a flow diagram showing a more detailed example of the compression method of figure 1.
Figure 2 is a diagram showing an example of a system for implementing one or more of the compression methods described herein.
Figure 2A is a diagram showing another example of a system for implementing a compression method described herein.
Figure 2B is a diagram showing another example of a system for implementing a compression method described herein.
Figure 3 is a schematic that shows a first example of a read that is globally mapped with a reference sequence.
Figure 4 is a schematic that shows a second example of a read that is globally mapped with a reference sequence, in a case where a fake mismatch has to be inserted.
Figure 5 is a diagram of an example of computing components that can be used to implement a system that executes the compression method of FIGs. 1 and 1A.
Figure 6 is a depiction of a plurality of bar graphs that show experimental results of the present disclosure.
Figure 7 is a depiction of a plurality of bar graphs that show additional experimental results of the present disclosure.
Figure 8 is a depiction of a plurality of bar graphs that show additional experimental results of the present disclosure.

### Detailed Description

The genomic sequences referred to by the present disclosure include, for example, and not as a limitation, nucleotide sequences, deoxyribonucleic acid (DNA) sequences, Ribonucleic acid (RNA), and amino acid sequences. Although the present disclosure is described herein in considerable detail with respect to genomic information in the form of a nucleotide sequence, it will be understood that the compression method according to the invention can be implemented for other genomic sequences as well, albeit with a few variations, as will be understood by a person skilled in the art.

Genome sequencing information is generated by sequencing machines in the form of sequences of nucleotides (or, more generally, bases) represented by strings of letters from a defined vocabulary. The smallest vocabulary is represented by five symbols: {A, C, G, T, N} representing the 4 types of nucleotides present in DNA namely Adenine, Cytosine, Guanine, and Thymine. In RNA Thymine is replaced by Uracil (U). N indicates that the sequencing machine was not able to call any base and so the real nature of the position is undetermined. Thus, for purposes of the present disclosure, the symbol "N" refers to an undetermined base and a number of "N" in a read refers to a number of undetermined bases in the read.

The nucleotide sequences produced by sequencing machines can be called "reads". Sequence reads can be between a few dozens to several thousand nucleotides long. Some technologies produce sequence reads in pairs where a first read of the pair is from one DNA strand and a second read of the pair is from another DNA strand. Throughout this disclosure, a "reference sequence" is any sequence to which reads comprised of nucleotides or base sequences produced by sequencing machines can be aligned/mapped. One example of such a reference sequence could actually be a reference genome, i.e. a sequence assembled by scientists as a representative example of a species' set of genes. However, a reference sequence could also consist of a synthetic sequence conceived to merely improve the compressibility of the reads in view of their further processing.

In some instances, sequencing machines can introduce errors in the sequence reads, and notably a use of a wrong symbol (i.e. representing a different nucleic acid) to represent the nucleic acid or base actually present in the sequenced sample. This type of substitution error may end up being identified as a "mismatch" by a mapping and aligning module. This is because the substitution error in a read may not match a corresponding location of a reference sequence when the read is aligned to the reference sequence. However, the meaning of "mismatch" is not limited to such scenarios. Instead, a "mismatch" can be any base or nucleotide of a read called by a sequencing device that does not match a corresponding location of a reference sequence when the read is aligned to a reference sequence with a threshold level of accuracy. Such mismatches can include candidate variants, variants, or other differences between an aligned read and a reference sequence location.

The present disclosure is directed towards a reference-based compression method that receives reads of sequences of nucleotides or bases as inputs, such reads having been previously aligned to a reference sequence by a mapping and aligning module, thereby creating aligned reads. In some implementations, the previously aligned reads can include reads that have been aligned using a software mapping and aligning module that performs mapping and aligning of the received reads to a reference sequence. For example, in some implementations, the software mapper can perform hash table based mapping and aligning of the received reads by executing software instructions using one or more processors such as one or more central process units (CPUs), one or more graphical processing units (GPUs), or any combination thereof. In other implementations, the previously aligned reads can include reads that have been aligned using a hardware mapping and aligning module that performs mapping and aligning of the received reads to a reference sequence. For example, in some implementations, the hardware mapping and aligning module can perform hash table based mapping and aligning by using one or more hardware processors such as one or more field-programmable gate arrays (FPGAs) having hardwired digital logic circuit configured to perform the hash table based mapping and aligning of the received reads.

The aligned reads are then stored as a list of reads in an initial file. The way to align reads and to store them once aligned in an initial file is not critical to the invention and is not the purpose of the present disclosure. Each read is then encoded as a position on the reference sequence and a list of differences with said reference sequence. Each read can then be reconstructed from the alignment encoded information and the reference sequence, by a proper decompression software configured as described herein by the present disclosure.

In some implementations, the a compression module of the present disclosure can be implemented via execution of software instructions by one or more CPUs or GPUs, execution of hardwired digital logic circuits of one or more hardware processors, or a combination of both, to process and compress aligned reads. The reads can be aligned to a reference sequence prior to compression of the reads without taking into account certain types of errors introduced in the sequence reads such as for example insertion errors or deletion errors. An insertion error consists in the insertion in one sequence read of one or more additional symbols that do not refer to any actually present nucleic acid. A deletion error consists in the deletion from one sequence read of one or more symbols that represent nucleic acids that are actually present in the sequenced sample. More precisely, in case of an insertion error or a deletion error in a given sequence read, the alignment software will then consider the resulting erroneous nucleic acids as substitution errors, also called "mismatches". This preferential choice for the alignment software configuration allows faster subsequent coding, providing notably a better compromise between speed and compression ratio.

For each aligned read, the mapping and aligning module can generate and provide a read record. In some implementations, each read record can be provided directly as an input to the compression module from the mapping and aligning module. In other implementations, each read record generated by the mapping and aligning module can be output and stored in a memory or other storage device. In such implementations, the compression module can later access the stored read records and compress the stored read records.

Each read record generated, provided, or stored by the mapping and aligning module includes data generated by the mapping and aligning module that describes the read represented by the read record. Such read records can include at least the following information: the absolute starting position of the aligned read with respect to the reference sequence, the length of the read, the type of alignment of the read such as whether the read is a mapped read or an unmapped read, the number of mismatches identified in the read, an indication as to whether the read is a perfectly mapped read or an imperfectly mapped read, the relative position of said possible mismatches in the read, or the like.

Though the example described here indicates that the data in the read record, and the data included therein, is generated by the mapping and aligning module, the present disclosure is not so limited. Instead, other intermediary modules between the mapping and aligning module and the compression module can be used to generate the read record, the data contained therein.

In some implementations, read records provided or stored by the mapping and aligning module can be provided or stored in a manner that preserves the sequential ordering of the read records generated by the mapping and aligning module. In some implementations, for example, each read record can also include data that indicates the read records placement in a sequential ordering of read records. Such data indicating the read records placement can include, for example, a sequence_id. In some implementations, this sequence_id can be, for example, a number that is begins with "1" for a first read record produced by the mapping and aligning module that is then incremented for each subsequent read record generated by the mapping and aligning module. The compression module of the present disclosure can then access these read records and compress these read records in their current sequential order without the need to reorder the read records into clusters of read records for compression. Compressing the read records in a manner that preserves their initial ordering as generated by the mapping and aligning module provides advantages over conventional methods by enabling lossless compression of the read records - since even the sequential ordering of the read records is preserved. In addition, preserving the order of the read records during compression also makes validation of read record compression easier.

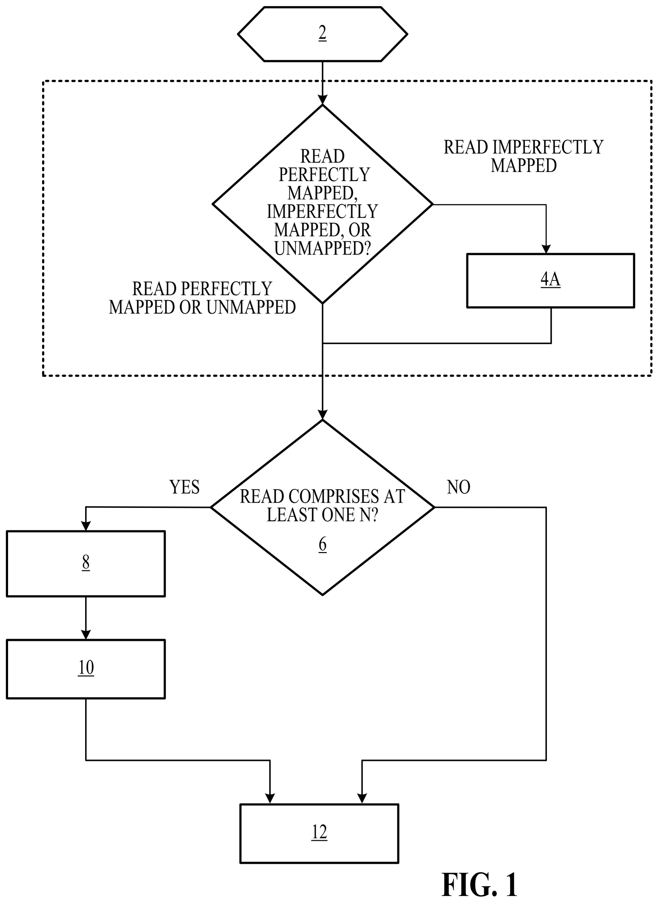

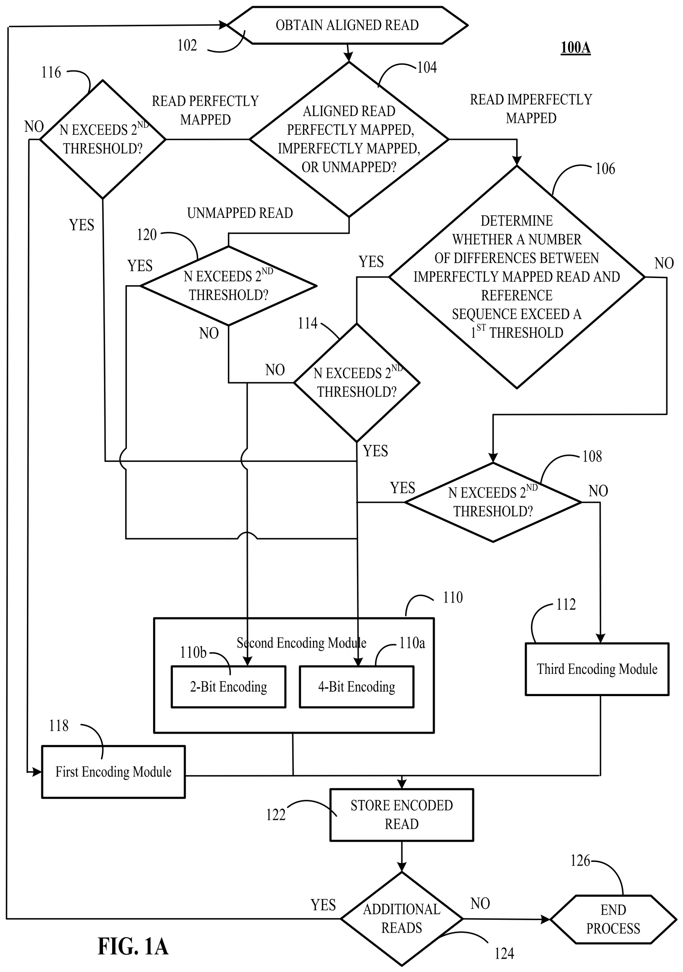

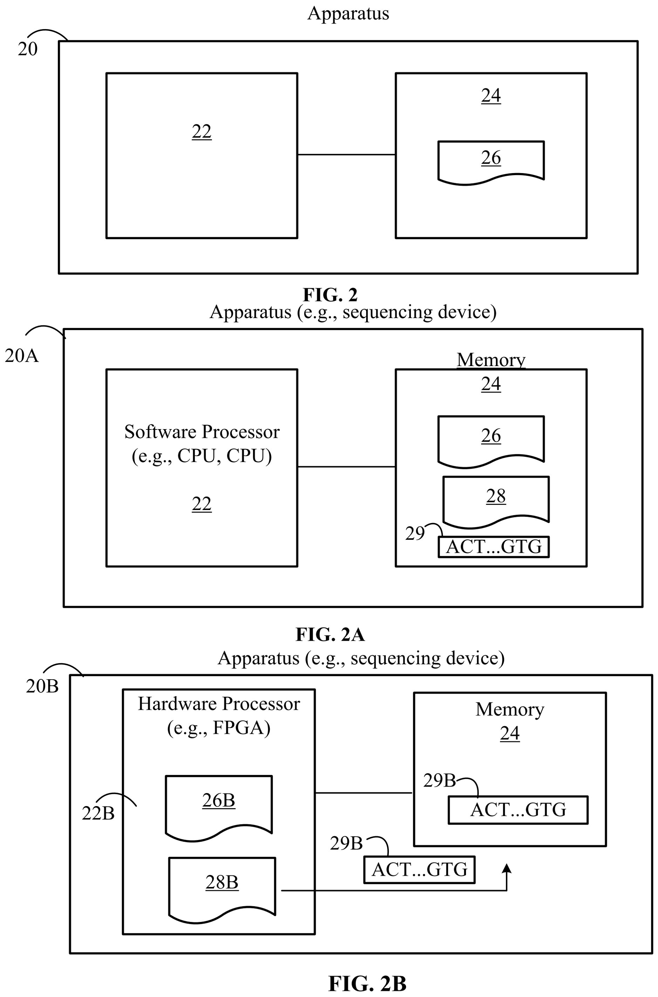

The compression method of the present disclosure will now be described with reference to Figure 1. In some implementations, for example, the method can be performed by an apparatus 20 shown in Figure 2. The apparatus 20 can include at least one processor 22 and at least one memory 24 operatively coupled to the at least one processor 22 to form a computing device. The memory 24 may store a computer program code or software 26 comprising computer executable instructions which, when executed by the processor 22, cause the processor 22 to perform operations of a compression module comprising execution of the stages of one or more of the compression methods described herein. However, the present disclosure need not be limited to being implemented by the apparatus 20.

For example, in some implementations, the compression methods of the present disclosure can be implemented by an apparatus 20A shown in figure 2A. The apparatus 20A is similar to the apparatus 20 in that the apparatus 20A also includes a processor 22 and at least one memory 24 operatively coupled to the at least one processor 22 to form a computing device. The memory 24 of apparatus 20A also stores computer program code or software 26 comprising computer executable instructions which, when executed by the processor 22, cause the processor 22 to perform operations comprising the stages of one or more of the compression methods described herein. In addition, however, the apparatus 20A also includes computer program code or software 28 comprising computer executable instructions which, when executed by the processor 22, cause the processor 22 to perform operations to realize functionality of a mapping and aligning module. The mapping and aligning module, whose functionality is realized via execution of computer software instructions, can generate one or more aligned reads 29 and store the aligned reads 29 in the memory 24. Then, the processor 22 can execute the software instructions 26 of a compression module to access one or more of the aligned reads 29 and compress the one or more aligned reads 29 using the stages of one or more compression methods described herein. In some implementations, the apparatus 20A can be a nucleic acid sequencing device.

By way of another example, in some implementations, the compression methods of the present disclosure can be implemented by an apparatus 20B shown in figure 2B. The apparatus 20B is different than the apparatus 20 in that the apparatus 20B includes one or more hardware processors 22B such as one or more field programmable gate arrays (FPGAs). In this example, the one or more hardware processors can realize functionality of the stages of one or more compression methods described herein and a mapping and aligning module in hardware circuitry of the one or more hardware processors 22B. For example, the hardware processor 22B can include hardwired digital logic circuits 26B configured as a compression module to perform the stages of one or more of the compression methods described herein. Likewise, the hardware processor 22B can include hardwired digital logic circuits 28B configured to perform operations of a mapping and aligning module that is configured to generate aligned read 29B and store the aligned read 29B in the memory 24. The digital hardwired logic circuits 26B configured as a compression module to realize the functionality of stages of one or more of the compression methods described herein can access the aligned read 29B from the memory 24, and compress the aligned read 29B using the compression methods described herein. In some implementations, the apparatus 20B can be a nucleic acid sequencing device. The initial file in which the aligned read records are stored as a list of reads is for example stored in a memory of the apparatus 20. In some implementations, the list of reads can include a plurality of aligned read records stored in the memory of the apparatus in a manner that preserves a sequence ordering of the read records as the produced by a mapping and aligning module. This sequence ordering of the aligned read records can be an order that is the same as that obtained at the end of the mapping and aligning stage.

In some implementations, the initial list of aligned reads can be divided into blocks of reads. For example, in some implementations, a list of aligned reads can be divided into blocks of 50 000 reads. However, this specific value of blocks of 50 000 reads should not be construed as limiting the scope of the present disclosure, as implementations of the present disclosure can be achieved in the same way using other values.

In some implementations, the blocks of reads can have the same block size. However, in other implementations, the blocks of reads may have varying block size. In any event, each block of reads can begin with a header containing information needed to decode the block, such as for example the size in bytes of the content of the block, and/or an identifier of the block or its content and/or the number of reads contained in the block. This allows support for the concatenation of compressed file, as well as streaming capabilities (each block of reads containing all the information needed to decode the reads of the block). Besides, since the compression method can then be performed block after block, this also allows multi thread processing on the blocks of reads, thereby allowing parallelization and some resulting gain in processing time. If all the reads of a given block have the same length, the read length is also stored in the header, otherwise a list of each read length is stored explicitly during the compression method.

Returning to Figure 1, the method preferably comprises an initial stage 2 where the apparatus obtains an aligned read record from a memory of the apparatus 20, 20A, or 20B. In some implementations, this can include accessing, by the apparatus, the memory or other storage device storing the plurality of read records in manner that preserves a sequence ordering of the read records as produced by a mapping and aligning module. For example, the apparatus may determine, based on a sequence_id of a previous read record and a sequene_id of one or more other read records store in the memory, a next read record for compression. In some implementations, the sequence_id can be numerical number that increments for each subsequent read record that was produced by the mapping and aligning module and the compression module can maintain a counter that increments upon each iteration of the compression process of FIG. 1 and provides an indication as to a next read record that should be accessed at stage 2.

Each read record contains information about the type of alignment of the read. Information about the type of alignment of a read can include any information that describes a level of mapping and alignment of the read to a reference genome. In some implementations, the types of alignment can include a perfect alignment an imperfect alignment, or an "unmapped" read alignment. A "perfect alignment" or a "perfectly mapped read" can include a read where each nucleotide of the read maps and aligns to a portion of the reference genome. In some implementations, a "perfect alignment" or "perfectly mapped read" can have zero mismatches and zero undetermined bases "N." In other implementations, a "perfect alignment" or "perfectly mapped" read can have zero mismatches, but potentially one or more undetermined bases "N." In general, the definition of an "imperfect alignment" or an "imperfectly mapped read" is dependent upon the meaning of "perfectly mapped read" implemented in a particular implementation of the compression methods described herein. If, for example, an implementation is used where a perfectly mapped read can contain zero mismatches and zero undetermined bases N, then an "imperfect alignment" or "imperfectly mapped read" means any read that matches at least a portion of the reference sequence and includes at least one mismatch or at least one N. However, if, for example, an implementations is used where a perfectly mapped read can contain zero mismatches by one or more N, then an "imperfect alignment" or "imperfectly mapped read" means any read having at least one mismatch other than an undisclosed base N, while at least a portion of the read matches a portion of the reference sequence (according other this definition of an imperfectly mapped read, an imperfectly mapped read may contain one or more N, provided it also contains one or more other mismatches). Thus, how any particular system or implementation is configured to recognize perfectly mapped reads will determine the meaning of imperfectly mapped reads for that implementation. An "unmapped read" can include a read that has not been mapped or aligned to a reference genome.

In some implementations, each read record include a plurality of bit flags that describe attributes of the read. In some implementations, the plurality of bit flags can be stored using one or more fields at the beginning of the read record. However, in other implementations, other fields of the read record can be used to store the plurality of bit flags. Each bit flag of the plurality of bit flags can use one of a plurality of values to indicate a value of its corresponding read attribute. In some implementations, the following bit flags can be used to indicate values of read attributes for a read record:
- a first bit flag indicative of a forward or reverse orientation versus the reference sequence,
- a second bit flag indicative of a perfect alignment or not,
- a third bit flag indicative of whether the read contains at least one N,
- a fourth bit flag indicative of whether the position information is encoded on 16 bits or 32 bits,
- a fifth bit flag indicative of whether the read is mapped or unmapped.

The following stages 4-12 are performed for each read of a plurality of reads. If the reads are grouped into blocks, then stages 4-12 are performed for each read of each block of reads.

The compression method of the present disclosure can include a next stage 4 of determining by the apparatus 20, 20A, or 20B, for each aligned read, whether said read is perfectly mapped with the reference sequence, imperfectly mapped with the reference sequence, or whether said read is unmapped with the reference sequence. In some implementations, the apparatus 20, 20A, 20B can determine whether the read is perfectly mapped, imperfectly mapped, or unmapped read based on information received from a mapping and aligning module. This information can include information such as, for example, whether the read represented by the obtained read record was mapped or unmapped, whether the read represented by the read record is perfectly mapped or imperfectly mapped, an indication of a number of total mismatches such as variants or sequencing errors, undetermined bases, or any combination thereof. In some implementations, this information can be included within the obtained read record itself.

In some implementations, the apparatus 20, 20A, or 20B may first determine whether the aligned read was mapped or unmapped. If the apparatus 20, 20A, or 20B determines that the aligned read was unmapped, then the apparatus can continue execution of the process of figure 1 at stage 6. Alternatively, if apparatus 20, 20A, or 20B determines that the read was mapped, then the apparatus 20, 20A, or 20B can determine if the read was imperfectly mapped or perfectly mapped.

In some implementations, the apparatus 20, 20A, or 20B can determine if a read was imperfectly mapped or perfectly mapped by evaluating a number of total number of mismatches in the read. In some implementations, this total number of mismatches can be provided by the mapping and aligning module and obtained from the obtained read record. In such implementations, if apparatus 20, 20A, or 20B determines that the total number of mismatches is equal to zero, then the apparatus 20, 20A, or 20B can determine at stage 4 that the obtained aligned read is a perfectly mapped read and can continue execution of the process of figure 1 at stage 6. Alternatively, if at stage 4, the apparatus 20, 20A, or 20B determines that the total number of mismatches is greater than zero, then the apparatus 20, 20A, or 20B can determine, at stage 4, that the read corresponding to the read record is an imperfectly mapped read and the apparatus 20, 20A, or 20B can continue execution of the process of figure 1 at stage 6.

However, it is noted that the above implementations are merely examples as to how the apparatus 20, 20A, or 20B can determine that an aligned read record is perfectly mapped, imperfectly mapped, or unmapped. For example, in some implementations, such a determination may be made based on information contained in the obtained read record and without comparison of a number of mismatches to a zero threshold. By way of example, the read record can maintain bit flags in the header, or other portion, of the read record that indicates whether the read is mapped or unmapped, perfectly mapped, imperfectly mapped, or the like. In such implementations, the apparatus 20, 20A, or 20B can made a determination as at stage 4 as to whether the aligned read record is mapped, unmapped, perfectly mapped, or imperfectly mapped based on the bit flags of the obtained read record without comparison of a number of mismatches to a zero threshold. Other implementations also fall within the scope of the present disclosure. For example, it is conceivable that implementations can be employed where information that is stored in a data structure that is different than the obtained read record can be accessed and considered to read bit flags, or other data, to indicate whether a particular read record is mapped, unmapped, perfectly mapped, or imperfectly mapped.

In some implementations, this determining step 4 can further include, for each imperfectly mapped read, comparing 4a the number of mismatches between said read and the reference sequence to a threshold value. This can include a total number of mismatches, with the total number of mismatches including a summation of any difference between the aligned read and the reference sequencing including variants, sequencing errors, and undetermined bases N. In some implementations, the number of mismatches can be provided by the mapping and aligning module and obtained from the read record.

In some implementations, the threshold value can be 31. This specific value can be chosen so as to provide the best possible compromise for storing the number of mismatches in a sufficiently compact manner, as will be better understood later with regard to stage 12. Indeed, it has been statistically observed that in a vast majority of the cases, the imperfectly mapped reads have less than 31 mismatches. The principle lying behind that choice consists in encoding in the most compact way the most frequent cases, leave to have some very few degraded cases. However, while using a threshold value of 31 mismatches in some implementations such as short read implementations where reads are approximately 150 nucleotides or bases in length can be advantageous, the present disclosure is not limited to only those implementations where the threshold value is equal to 31. Instead, for other implementations it may be desirable to use of a higher threshold value than 31. For example, while aspects (e.g., threshold value of 31 mismatches) may be intended for use compressing read records representing reads generated by short read sequencers, it is contemplated that the genomic data compression methods of the present can be used in other implementations such as to compress read records generated by long read sequencers. Thus, in such implementations, where reads are represented by read records that are significantly longer than 150 nucleotides or bases in length, the threshold value can be set to a higher value than 31 to enable functionality of the compression methods of the present disclosure for long read systems.

If a read is determined to be imperfectly mapped with a number of mismatches lower than the threshold value, the determining stage 4 can also include an additional determination as to whether the read is globally mapped or locally mapped with the reference sequence. A "globally mapped read" is an imperfectly mapped read whose whole sequence, comprising the beginning and the end of the read, is imperfectly mapped with the reference sequence. A "locally mapped read" is an imperfectly mapped read containing a segment of nucleotides or bases that is imperfectly mapped with the reference sequence. Said segment of nucleotides or bases thus corresponds to a portion of the initial read.

In some implementations, the compression method can further includes a stage 6 of determining, for each aligned read, whether said read comprises at least one undetermined base "N," i.e. whether said read comprises at least one mismatch corresponding to a case in which the sequencing machine was not able to call any base or nucleotide. The method then comprises, for each read comprising at least one "N," a stage 8 of determining the number of such undetermined bases "N" and a stage 10 of comparing said number of undetermined bases "N" with a reference threshold value. In some implementations, the reference threshold value can equal to 31. However, in other implementations, other the reference thresholds can be set to other values.

Whatever the outcome of the determination stage 4, the method comprises a next stage 12 of encoding the reads according to said determination. More precisely, the reads that are determined to be perfectly mapped with the reference sequence, whether they comprise no undetermined base "N" or has a number of undetermined bases "N" lower than the reference threshold value, are encoded according to a first encoding process. The reads that are determined to be unmapped or the reads that are determined to be perfectly mapped but with a number of undetermined bases "N" greater than the reference threshold value are encoded according to a second encoding process in which each nucleotide or base is individually encoded, regardless of whether said nucleotide or base is aligned or not. The reads that are determined to be imperfectly mapped are encoded according to the second encoding process or to a third encoding process. More precisely, the reads that are determined to be imperfectly mapped with a number of mismatches greater than the threshold value are encoded according to the second encoding process. If a read is determined to be imperfectly mapped with a number of mismatches lower than the threshold value, if said read comprises no N or has a number of N lower than the reference threshold value, then said read is encoded according to the third encoding process. If not, i.e. if the read has a number of N greater than the reference threshold value, then said read is encoded according to the second encoding process.

Whether a given read has been determined as being perfectly mapped, imperfectly mapped or unmapped, if said read comprises at least one N but has a number of N lower than the reference threshold value, the encoding stage 12 comprises encoding a list of positions along the reference sequence, said positions corresponding to the positions of the N in the reference sequence. The list of positions is then stored in a memory of a computing device, said device implementing the compression method. If a read comprises at least one N but has a number of N lower than the reference threshold value, and is to be encoded according to the second encoding process, then each nucleotide or base of the read is individually encoded on 2 bits.

If a read comprises at least one N but with a number of N greater than the reference threshold value, then said read is in any case encoded according to the second encoding process, and each nucleotide or base of the read is individually encoded on 4 bits. In this case, the encoding stage 12 does not comprise encoding and storing a list of positions of the N in the reference sequence. Indeed, each N mismatch is then directly encoded according to the second encoding process, in the very same way as the other nucleotides or bases of the read.

The first and third encoding processes comprise distinct sets of descriptors. Each set of descriptors univocally represents the reads associated to the corresponding encoding process, each of the first and third encoding processes being a reduced information entropy encoding process. More precisely, the third encoding process comprises a first encoding subprocess and a second encoding subprocess. The imperfectly mapped reads that are determined to be globally mapped during stage 4 are encoded according to the first encoding subprocess. The imperfectly mapped reads that are determined to be locally mapped during stage 4 are encoded according to the second encoding subprocess. The first and second encoding subprocesses comprise distinct sets of descriptors, each set of descriptors univocally representing the reads associated to the corresponding encoding subprocess.

The alignment information encoded for each read, and which enables the reconstruction of the whole read sequence during the decompression of the data, then depends on the corresponding encoding process or subprocess used for said read.

For example, in some implementations, a first set of descriptors used for the first encoding process can include:
∘ the absolute starting position of the perfectly mapped read with respect to the reference sequence (encoded on 16 or 32 bits), and
∘ the length of the read (encoded with differential coding relative to the length of the previous read, with variable length code ranging from 2 bits to 34 bits).

By way of another example, in some implementations, a second set of descriptors used for the first encoding subprocess can include:
∘ the absolute starting position of the imperfectly mapped read with respect to the reference sequence (encoded on 16 or 32 bits),
∘ the length of the read (encoded with differential coding relative to the length of the previous read, with variable length code ranging from 2 bits to 34 bits), and
∘ a list of the mismatches of the read.

By way of another example, in some implementations, a third set of descriptors used for the second encoding subprocess can include:
∘ the absolute starting position of the imperfectly mapped portion of the read with respect to the reference sequence - also called local alignment starting position (encoded on 16 or 32 bits),
∘ the length of the read (encoded with differential coding relative to the length of the previous read, with variable length code ranging from 2 bits to 34 bits),
∘ a list of the mismatches of the read, and
∘ the length of the clipped portions of the read that are not part of the alignment (encoded on 8 bits for each clipped portion).

Preferably, the list of mismatches which is encoded in the first and second subprocesses can include a header. For example, in some implementations, the header can be encoded using a bit flag and be encoded on one byte. In such implementations, the five first bits of the one-byte header can be used to encode the number of mismatches contained in the read. In implementations where the threshold value is equal to 31, the number of mismatches can range between 0 and 31. One bit of the one-byte header can be used to encode whether the imperfectly mapped read is globally or locally mapped. Another bit of the one-byte header can be used to encode whether or not the 2-bit mode is activated for the second encoding process. The last bit of the one-byte header can be used to encode whether or not the 4-bit mode is activated for the second encoding process. In some implementations, for each read encoded according to the second encoding subprocess during the encoding stage 12, the clipped portions of said read (i.e. those portions that are not part of the local alignment) are concatenated, and each nucleotide or base of said clipped portions is individually encoded. In some implementations, each nucleotide or base of such clipped portions of the read is individually encoded on 2 bits.

In some implementations, each mismatch encoded in the list of mismatches of an imperfectly mapped read (i.e. encoded according to the first or second encoding subprocess) can be encoded on 1 byte. More precisely, each mismatch of an imperfectly mapped read that is to be encoded according to the first or second encoding subprocess may be encoded as follows:
∘ the two first bits of the byte are used to encode the alternate nucleotide or base present in the read instead of the corresponding reference nucleotide or base in the reference sequence,
∘ the six last bits are used to encode the position of the mismatch in the reference sequence, said position being computed as an offset from the previous mismatch of the read. This computed position can be a relative position of the mismatch, except for the first mismatch of the read for which the absolute position is encoded. The range of this offset, which is encoded on 6 bits, can therefore be [0-63].

The encoded, or compressed, record that result from the completion of the process of figure 1 can be stored in a memory or other storage device of the apparatus. In some implementations, this encoded, or compressed, record can be stored in the memory or other storage device of the apparatus in a manner that maintains the sequence ordering of the read records. This helps to ensure that compression of the aligned read records is loss-less since even the initial sequence ordering of the aligned read records is preserved.

### Obtain an aligned read record at stage 102

The compression method of FIG. 1 is described in more detail with reference to compression method 100A of FIG. 1A. Execution of the compression method 100A by an apparatus 20, 20A, or 20B can begin with an initial stage 102 that includes obtaining an aligned read record (also referred to below as "obtained read record" or "unmapped read" / "mapped read" / "perfectly mapped read" / "imperfectly mapped read" based on the obtained read record's subsequent classification during execution of method 100A). In some implementations, the aligned read record can be obtained from a plurality of aligned read records that are stored in a manner so that their initial order as provided by the sequencing device is preserved. Thus, the entire operation of the mapping and aligning module and the compression module can keep the read records in their initial order as provided by the sequencing device. In some implementations, the aligned read records can be stored to preserve their initial order by using a sequence_id that is stored with each aligned read record and incremented with each aligned read record produced by the mapping and aligning module.

Determine whether a read corresponding to the aligned read record is perfectly mapped, imperfectly mapped, or unmapped at stage 104

The compression method of the present disclosure can include a next stage 104 of determining by the apparatus 20, 20A, or 20B whether the obtained read record corresponds to a read that is perfectly mapped with the reference sequence, imperfectly mapped with the reference sequence, or unmapped with the reference sequence. In some implementations, the apparatus 20, 20A, 20B can determine whether the read is perfectly mapped, imperfectly mapped, or unmapped read based on information received from a mapping and aligning module. This information can include information such as, for example, whether the read represented by the obtained read record was mapped or unmapped, whether the read represented by the read record is perfectly mapped or imperfectly mapped, an indication of a number of total mismatches such as variants or sequencing errors, undetermined bases, or any combination thereof. In some implementations, this information can be included within the read record itself.

In some implementations, the apparatus 20, 20A, or 20B may first determine at stage 104 whether the aligned read was mapped or unmapped. If the apparatus 20, 20A, or 20B determines that the aligned read was unmapped, then the apparatus can continue execution of the process 100A of figure 1A at stage 120. Alternatively, if apparatus 20, 20A, or 20B determines that the read was mapped, then the apparatus 20, 20A, or 20B can further determine during stage 104 if the read was imperfectly mapped or perfectly mapped.

In some implementations, the apparatus 20, 20A, or 20B can determine during stage 104 if a read was imperfectly mapped or perfectly mapped by evaluating a number of a number of mismatches in the read. In some implementations, the number of mismatches can be provided by the mapping and aligning module and obtained from the read record. The number of mismatches may be tallied in different ways for different implementations. In some implementations, the number of mismatches at stage 104 may not include a number of undetermined bases N. In other implementations, the number of mismatches determined at stage 104 may include a total of the number of mismatches and a number of undetermined bases N.

In the example of figure 1A, it is assumed that an undetermined base N is not a mismatch. As a result, a perfectly mapped read may include 0 mismatches and one or more undetermined bases N. Thus, an imperfectly mapped read, in this implementation, would need to have at least one mismatch, and may or may not have any undetermined bases N. However, in other implementations, the process of figure 1A can be modified by assuming that the presence of an N in a read could be a mismatch. In such implementations, a read could be determined to be a perfectly mapped read only if the read is determined to have 0 mismatches and 0 undetermined bases N, with a read having 0 mismatches and one or more undetermined bases N being classified as an imperfectly mapped read.

In a first implementation, at stage 104, if apparatus 20, 20A, or 20B determines that the total number of mismatches is equal to zero and the total number of undetermined bases N is zero or more, then the apparatus 20, 20A, or 20B can determine at stage 4 that the obtained aligned read is a perfectly mapped read and can continue execution of the process 100A of figure 1A at stage 116. Alternatively, in this first implementation if during stage 104, the apparatus 20, 20A, or 20B determines that the total number of mismatches is greater than zero and the total number of undetermined bases N is zero or more, then the apparatus 20, 20A, or 20B can determine, during stage 104, that the read corresponding to the obtained read record is an imperfectly mapped read and the apparatus 20, 20A, or 20B can continue execution of the process 100A of figure 1A at stage 106.

In a second and alternative implementation, at stage 104, the apparatus 20, 20A, or 20B will only determine that the read is a perfectly mapped read if the total number of mismatches is equal to zero and the total number of undetermined bases N is zero, and in such a scenario, the apparatus 20, 20A, or 20B can can continue execution of the process 100A of figure 1A at stage 116. Alternatively, in this second implementation if during stage 104, the apparatus 20, 20A, or 20B determines that the total number of mismatches is greater than zero or the total number of undetermined bases N is greater than zero, then the apparatus 20, 20A, or 20B can determine, during stage 104, that the read corresponding to the obtained read record is an imperfectly mapped read and the apparatus 20, 20A, or 20B can continue execution of the process 100A of figure 1A at stage 106.

However, it is noted that the above implementations are merely examples as to how the apparatus 20, 20A, or 20B can determine at stage 104 that a read corresponding to the obtained read record is perfectly mapped, imperfectly mapped, or unmapped. For example, in some implementations, such a determination can instead be made based on information contained in the obtained read record and without comparison of a number of mismatches to a threshold, without a comparison of a number of undetermined bases N to a threshold, or both. By way of example, the read record can maintain bit flags in the header, or other portion, of the read record that indicates whether the read is mapped or unmapped, perfectly mapped, imperfectly mapped, or the like. In such implementations, the apparatus 20, 20A, or 20B can made a determination as at stage 4 as to whether the aligned read record is mapped, unmapped, perfectly mapped, or imperfectly mapped based on the bit flags of the read record without comparison of a number of mismatches or undisclosed bases N to thresholds. Other implementations also fall within the scope of the present disclosure. For example, it is conceivable that implementations can be employed where information that is stored in a data structure that is different than the read record can be accessed and considered to read bit flags, or other data, to indicate whether a read corresponding to the particular read record is mapped, unmapped, perfectly mapped, or imperfectly mapped.

### "Read Imperfectly Mapped" branch of stage 104

If the apparatus 20, 20A, or 20B determines at stage 104 that the read corresponding to the obtained read record is an imperfectly mapped read, then the apparatus 20, 20A, or 20B can determine, at stage 106, whether a number of differences between said imperfectly mapped read and the reference sequence exceeds a first threshold value. This can include a total number of mismatches, with the total number of mismatches including a summation of any difference between the aligned read and the reference sequence including variants, sequencing errors, and undetermined bases N. In other implementations, the number differences at stage 106 may include only a number of mismatches without factoring in the number of undetermined bases N. In some implementations, the number of mismatches can be provided by the mapping and aligning module and obtained from the read record.

In some implementations, the first threshold value can be 31. This specific value can be chosen so as to provide the best possible compromise for storing the number of mismatches in a sufficiently compact manner, as will be better understood later with regard to subsequent stages. Indeed, it has been statistically observed that in a vast majority of the cases, the imperfectly mapped reads have less than 31 mismatches. The principle lying behind that choice consists in encoding in the most compact way the most frequent cases, leave some very few degraded cases. However, though there are particular advantages that can be achieved using a first threshold value of 31, the present disclosure is not limited to only those implementations where the first threshold value is equal to 31. Instead, for other implementations it may be desirable to use of a higher threshold value than 31. For example, while aspects (e.g., threshold value of 31 mismatches) may be intended for use compressing read records representing reads generated by short read sequencers, it is contemplated that the genomic data compression methods of the present can be used in other implementations such as to compress read records generated by long read sequencers. Thus, in such implementations, where reads are represented by read records that are significantly longer than 150 nucleotides or bases in length, the threshold value can be set to a higher value than 31 to enable functionality of the compression methods of the present disclosure for long read systems.

### "YES" branch of stage 106

If the apparatus 20, 20A, or 20B determines at stage 106 that the number of differences between the imperfectly mapped read and the reference sequence exceeds the first threshold, then the apparatus can continue execution of the process 100A at stage 114. At stage 114, the apparatus 20, 20A, or 20B can determine whether a number of undetermined bases "N" in the imperfectly mapped read exceeds a second threshold value. In some implementations, the second threshold value can also be equal to 31. However, like the first thresholds, the second threshold value of the present disclosure is not limited to a value of 31. Instead, any number value, including higher values than 31, can be used for the second threshold value based on the length of reads at issued in the implementation. Moreover, there is no requirement that the first threshold and the second threshold use the same threshold value.

### "YES" branch of stage 114

If it is determined by the apparatus 20, 20A, or 20B that the number of undisclosed bases "N" in the imperfectly mapped read exceeds the second threshold, then the apparatus 20, 20A, or 20B can determine that the imperfectly mapped read is to be encoded using the second encoding module 110 to encode the imperfectly mapped read using the second encoding process. The second encoding process is the same as the second encoding process described above with respect to figure 1, in which each nucleotide or base is individually encoded, regardless of whether said nucleotide or base is aligned or not. In some implementations, because the apparatus 20, 20A, or 20B determined that the number of undetermined bases "N" exceeded the second threshold at stage 114, the apparatus 20, 20A, or 20B can use the second encoding module to encode the read into 4 bits 110a using the second encoding process. Once read is encoded using the second encoding process 110 using 4-bit encoding 110a, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

### "NO" branch of stage 114

If the apparatus 20, 20A, or 20B determines, during stage 114, that the number of undisclosed bases "N" in the imperfectly mapped read does not exceed the second threshold, then the apparatus 20, 20A, or 20B can determine that the imperfectly mapped read is to be encoded using the second encoding module 110 to encode the imperfectly mapped read using the second encoding process. The second encoding process is the same as the second encoding process described above with respect to figure 1, in which each nucleotide or base is individually encoded, regardless of whether said nucleotide or base is aligned or not. In some implementations, because the apparatus 20, 20A, or 20B determined that the number of undetermined bases "N" did not exceed the second threshold at stage 114, the apparatus 20, 20A, or 20B can use the second encoding module to encode the read into 2 bits 110b using the second encoding process. Once read is encoded using the second encoding process 110 using 2-bit encoding 110b, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

### "NO" branch of stage 106

If the apparatus 20, 20A, or 20B determines at stage 106 that the number of differences between the imperfectly mapped read and the reference sequence does not exceed a first threshold, then the apparatus 20, 20A, or 20B can continue execution of the process 100A at stage 108. At stage 108, the apparatus 20, 20A, or 20B can determine whether the imperfectly mapped read includes more than a second threshold number of undetermined bases "N."

### "YES" branch of stage 108

If the apparatus 20, 20A, or 20B determines, at stage 108, that the number of undisclosed bases "N" in the imperfectly mapped read exceeds the second threshold, then the apparatus 20, 20A, or 20B can determine at stage 108 that the imperfectly mapped read is to be encoded using the second encoding module 110 to encode the imperfectly mapped read using the second encoding process. The second encoding process is the same as the second encoding process described above with respect to figure 1, in which each nucleotide or base is individually encoded, regardless of whether said nucleotide or base is aligned or not. In some implementations, because the apparatus 20, 20A, or 20B determined that the number of undetermined bases "N" exceeded the second threshold at stage 108, the apparatus 20, 20A, or 20B can use the second encoding module to encode the read into 4 bits 110a using the second encoding process. Once read is encoded using the second encoding process 110 using 4-bit encoding 110a, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

### "NO" branch of stage 108

If the apparatus 20, 20A, or 20B determines, during stage 108, that the imperfectly mapped read includes a number of undetermined bases "N" that does not satisfy the second threshold, then the apparatus 20, 20A, or 20B can use the third encoding module 112 to encode the imperfectly mapped read using the third encoding process. The third encoding process in figure 1A is the same as the third encoding process described above with reference to the process of figure 1 and uses the same descriptors as the third encoding process described above. Once the read is encoded using the third encoding process of the third encoding module 112, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

### "Read Perfectly Mapped" branch of stage 104

Alternatively, if it is determined at stage 104 that the read corresponding to the obtained read record is a perfectly mapped read, then the apparatus 20, 20A, or 20B can determine, at stage 116, whether the perfectly mapped read includes a number of undetermined bases "N" that exceeds a second threshold value. In some implementations, the second threshold value can also be equal to 31. However, like the first thresholds, the present disclosure is not limited to a second threshold value of 31. Instead, any number value, including higher values than 31, can be used for the second threshold value based on the length of reads at issued in the implementation. Moreover, there is no requirement that the first threshold and the second threshold use the same threshold value.

### "NO" branch of stage 116

If the apparatus 20, 20A, or 20B determines at stage 116 that the perfectly mapped read does not include more than a second threshold number of undetermined bases "N," then the apparatus 20, 20A, or 20B can determine to encode the read using the first encoding module 122 using a first encoding process. If the perfectly mapped read does not include any undetermined bases "N," then the first encoding module 122 executes a first encoding process that is the same as the first encoding process described above with reference to figure 1 and uses the same descriptors as the first encoding process described above. Alternatively, if the perfectly mapped read includes one or more "N," then the first encoding module 122 encodes the perfectly mapped read using the first encoding process described above with reference to figure 1 and using the same descriptors for the first encoding process described above. In addition, in the particular implementation where the perfectly mapped read include one o more N (but less than a second threshold number of N), the first encoding module 118 can also store a list of positions on the read for the undetermined bases N.

Once read is encoded using the first encoding module 118, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

### "YES" branch of stage 116

However, if the apparatus determines at stage 116 that the read does include more than a second threshold number of undetermined bases "N," then the apparatus 20, 20A, or 20B can use the second encoding module 110 to encode the read into 4 bits 110a using the second encoding process. The second encoding process of figure 1A is the same as the second encoding process described above with respect to figure 1, in which each nucleotide or base is individually encoded, regardless of whether said nucleotide or base is aligned or not. Once read is encoded using the second encoding process 110 using 4-bit encoding 110a, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

### "Unmapped Read" branch of stage 104

Alternatively, if it is determined at stage 104 that the read corresponding to the obtained read record is an unmapped read, then the apparatus 20, 20A, or 20B can determine, at stage 120, whether the unmapped read includes a number of undetermined bases "N" that exceeds a second threshold value. In some implementations, the second threshold value can also be equal to 31. However, like the first thresholds, the present disclosure is not limited to a second threshold value of 31. Instead, any number value, including higher values than 31, can be used for the second threshold value based on the length of reads at issued in the implementation. Moreover, there is no requirement that the first threshold and the second threshold use the same threshold value.

### "NO" branch of stage 120

If the apparatus 20, 20A, or 20B determines at stage 120 that the unmapped read does not include more than a second threshold number of undetermined bases "N," then the apparatus 20, 20A, or 20B can determine to encode the read using the second encoding module 110 using a second encoding process. The second encoding process is the same as the second encoding process described above with respect to figure 1, in which each nucleotide or base is individually encoded, regardless of whether said nucleotide or base is aligned or not. In some implementations, because the apparatus 20, 20A, or 20B determined that the number of undetermined bases "N" did not exceed the second threshold at stage 120, the apparatus 20, 20A, or 20B can use the second encoding module to encode the read into 2 bits 110b using the second encoding process. Once read is encoded using the second encoding process 110 using 2-bit encoding 110b, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

### "YES" branch of stage 120

However, if the apparatus determines at stage 120 that the unmapped read does include more than a second threshold number of undetermined bases "N," then the apparatus 20, 20A, or 20B can use the second encoding module 110 to encode the read into 4 bits 110a using the second encoding process. The second encoding process of figure 1A is the same as the second encoding process described above with respect to figure 1, in which each nucleotide or base is individually encoded, regardless of whether said nucleotide or base is aligned or not. Once the read is encoded using the second encoding process 110 using 4-bit encoding 110a, the apparatus 20, 20A, or 20B can store the encoded read in a memory or other storage device at stage 122. The apparatus 20, 20A, or 20B can determine, at stage 124, whether there is another, sequentially ordered aligned read that is to be compressed. And, if there is another, sequentially ordered aligned read that is to be compressed, the apparatus 20, 20A, or 20B can execute the operations of stage 102 in order to obtain the next sequentially ordered aligned read record and execute the process 100A again. The apparatus 20, 20A, or 20B than the continue to iteratively execute process 100A until no more sequentially ordered aligned read records are identified at stage 124. Upon such a determination, the process 100A can terminate at stage 126.

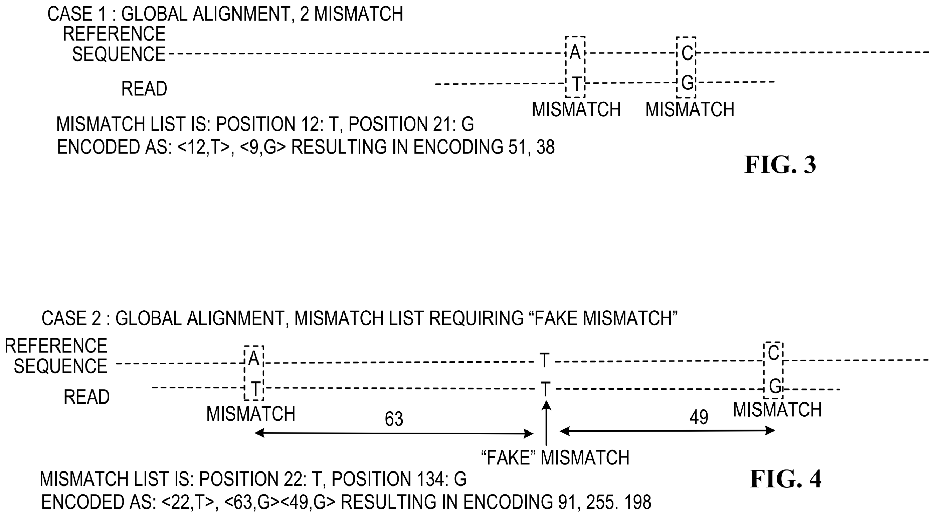

Figure 3 provides an example of the encoding of the mismatches of a read according to the first encoding subprocess. The read is an imperfectly mapped read, which is globally mapped with the reference sequence. The read has two mismatches:
∘ a first mismatch, located in the 12^{th} position in the read, which consists in a substitution of a A nucleotide in the reference sequence by a T nucleotide in the read, and
∘ a second mismatch, located in the 21 ^{th} position in the read, which consists in a substitution of a C nucleotide in the reference sequence by a G nucleotide in the read.

The list of the mismatches of the read is then encoded as:
∘ <12, T>, the value "12" corresponding to the absolute position of the first mismatch in the read, and
∘ <9, G>, the value "9" corresponding to the relative position of the second mismatch in the read, i.e. the offset between the second mismatch and the first mismatch.

<12, T> may for example be converted into the value "51" (encoded on 1 byte), and <9, G> may be converted into the value "38" (encoded on 1 byte). Such a byte encoding is obtained with:
offset position x 4 + nucleotide value (with A=0, C=1, G=2, T=3)

Preferably, for each imperfectly mapped read that is to be encoded according to the first or second encoding subprocess, if the offset computed between a given mismatch of the read and the previous mismatch is greater than a maximum encodable value, then at least one "fake" mismatch is inserted between said two mismatches until every offset between each of said mismatches and the at least one "fake" mismatch is lower than said maximum encodable value. A "fake" mismatch is defined as a mismatch for which the bits of the byte used to encode the mismatch encode a nucleotide or base that is equal to the corresponding reference nucleotide or base in the reference sequence. In some implementations, the maximum encodable value is equal to 63, corresponding to the maximum value that is encodable on 6 bits. However, the present disclosure is not limited to implementations have a maximum encodable value of 63. For implementations have a maximum encodable value of greater than 63, additional bits can be used to encode the value. In such implementations, this can require, for example, adjustment of other bit-lengths in the header for the read, an increase in header size beyond one byte, or a combination of both. Accordingly, features of the algorithm of the present disclosure are flexible for particular use cases but implementations that may arise from design changes may result in corresponding trade-offs in performance, which may be acceptable and even beneficial in certain circumstances of any particular implementation.

Figure 4 provides an example of the encoding of the mismatches of a read according to the first encoding subprocess, in a case where a "fake" mismatch has to be inserted. The read is an imperfectly mapped read, which is globally mapped with the reference sequence. The read has two mismatches:
∘ a first mismatch, located in the 22^{th} position in the read, which consists in a substitution of a A nucleotide in the reference sequence by a T nucleotide in the read, and
∘ a second mismatch, located in the 134 ^{th} position in the read, which consists in a substitution of a C nucleotide in the reference sequence by a G nucleotide in the read.

The position offset between the second and the first mismatches is of 112, which is greater than the maximum encodable value of 63. A "fake" mismatch therefore has to be inserted between the two mismatches, so that every offset between each of the mismatches and the "fake" mismatch is lower than said maximum encodable value. A "fake" mismatch with a T nucleotide (corresponding to a "real" T nucleotide in the reference sequence) is for example inserted in the 85^{th} position in the read. The position offset computed between the "fake" mismatch and the first mismatch is 63, which is corresponds to the maximum encodable value. The position offset computed between the second mismatch and the "fake" mismatch is of 49, which is lower than 63.

The list of the mismatches of the read is then encoded as:
∘ <22, T>, the value "22" corresponding to the absolute position of the first mismatch in the read,
∘ <63, T>, the value "63" corresponding to the relative position of the "fake" mismatch in the read, i.e. the offset between the "fake" mismatch and the first mismatch, and
∘ <49, G>, the value "49" corresponding to the relative position of the second mismatch in the read, i.e. the offset between the second mismatch and the "fake" mismatch.
<22, T> may for example be converted into the value "91" (encoded on 1 byte), <63, T> may be converted into the value "255" (encoded on 1 byte), and <49, G> may be converted into the value "198" (encoded on 1 byte). Such a byte encoding is obtained with:
offset position x 4 + nucleotide value (with A=0, C=1, G=2, T=3)

The method comprises a final step 14 of providing a compressed file comprising a list of encoded reads. The encoded reads are stored in the compressed file in the same order as that of the reads stored in the initial uncompressed file. Each read can then be reconstructed from the alignment encoded information and the reference sequence, by a proper decompression software and/or method configured according to the present invention.

Although described with reference to an exemplary architecture of a computing device 20 (shown in Figure 2 for illustrative purposes), the inventive techniques herewith disclosed may be implemented in hardware, software, firmware or any combination thereof. When implemented in software, the computer program code may be stored on a computer medium and executed by a hardware processing unit comprising one or more processors, as is the case with the device 20 of Figure 2. It should be understood that the term "processor" as used herein is intended to include one or more processing devices, including a signal processor, a microprocessor, a microcontroller, an application-specific integrated circuit (ASIC), a field programmable gate array (FPGA) or other type of processing circuitry, as well as portions or combinations of such circuitry elements. Also, the term "memory" as used herein is intended to include electronic memory associated with a processor, such as random access memory (RAM), read-only memory (ROM) or other types of memory, in any combination.

Accordingly, software instructions or code for performing the methodologies and protocols described herein may be stored in one or more of the associated memory devices, e.g., ROM, fixed or removable memory, and, when ready to be utilized, loaded into RAM and executed by the processor.

The techniques of this disclosure may be implemented in a wide variety of devices or apparatuses, including for example mobile phones, computers, servers, tablets and similar devices.

Although illustrative embodiments of the present invention have been described herein with reference to the accompanying drawings, it is to be understood that the invention is not limited to those precise embodiments, and that various other changes and modifications may be made by one skilled in the art without departing from the scope or spirit of the invention.

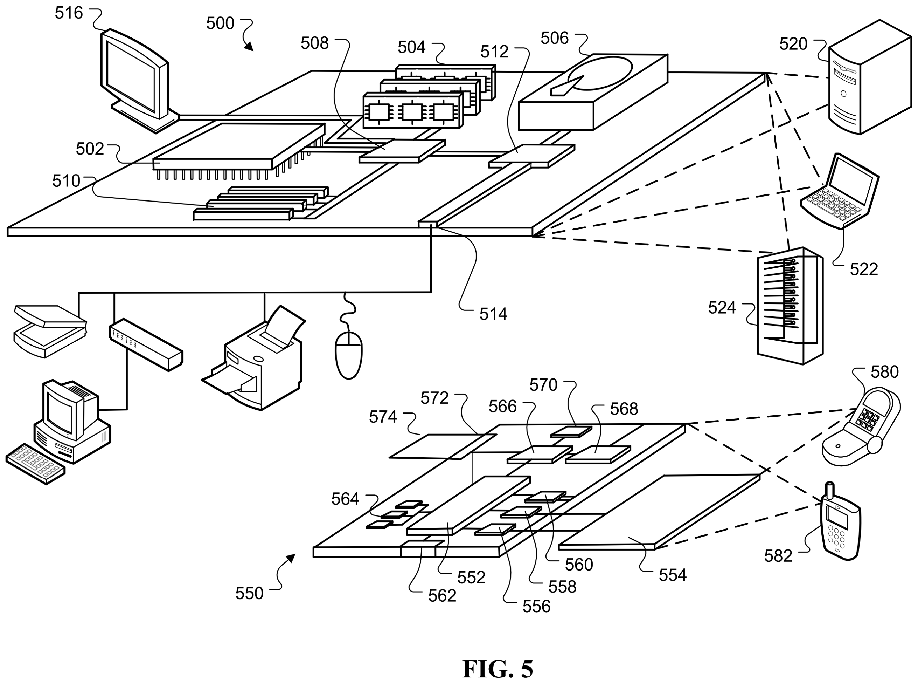

Figure 5 is a diagram of an example of computing components that can be used to implement a system that executes the compression method of FIGs. 1 and 1A.

Computing device 500 is intended to represent various forms of digital computers, such as laptops, desktops, workstations, personal digital assistants, servers, blade servers, mainframes, and other appropriate computers. Computing device 550 is intended to represent various forms of mobile devices, such as personal digital assistants, cellular telephones, smartphones, and other similar computing devices. Additionally, computing device 500 or 550 can include Universal Serial Bus (USB) flash drives. The USB flash drives can store operating systems and other applications. The USB flash drives can include input/output components, such as a wireless transmitter or USB connector that can be inserted into a USB port of another computing device. The components shown here, their connections and relationships, and their functions, are meant to be examples only, and are not meant to limit implementations of the inventions described and/or claimed in this document.

Computing device 500 includes a processor 502, memory 504, a storage device 508, a high-speed interface 508 connecting to memory 504 and high-speed expansion ports 510, and a low speed interface 512 connecting to low speed bus 514 and storage device 508. Each of the components 502, 504, 508, 508, 510, and 512, are interconnected using various busses, and can be mounted on a common motherboard or in other manners as appropriate. The processor 502 can process instructions for execution within the computing device 500, including instructions stored in the memory 504 or on the storage device 508 to display graphical information for a GUI on an external input/output device, such as display 516 coupled to high speed interface 508. In other implementations, multiple processors and/or multiple buses can be used, as appropriate, along with multiple memories and types of memory. Also, multiple computing devices 500 can be connected, with each device providing portions of the necessary operations, e.g., as a server bank, a group of blade servers, or a multi-processor system.

The memory 504 stores information within the computing device 500. In one implementation, the memory 504 is a volatile memory unit or units. In another implementation, the memory 504 is a non-volatile memory unit or units. The memory 504 can also be another form of computer-readable medium, such as a magnetic or optical disk.

The storage device 508 is capable of providing mass storage for the computing device 500. In one implementation, the storage device 508 can be or contain a computer-readable medium, such as a floppy disk device, a hard disk device, an optical disk device, or a tape device, a flash memory or other similar solid-state memory device, or an array of devices, including devices in a storage area network or other configurations. A computer program product can be tangibly embodied in an information carrier. The computer program product can also contain instructions that, when executed, perform one or more methods, such as those described above. The information carrier is a computer- or machine-readable medium, such as the memory 504, the storage device 508, or memory on processor 502.

The high-speed controller 508 manages bandwidth-intensive operations for the computing device 500, while the low speed controller 512 manages lower bandwidth intensive operations. Such allocation of functions is only an example. In one implementation, the high-speed controller 508 is coupled to memory 504, display 516, e.g., through a graphics processor or accelerator, and to high-speed expansion ports 510, which can accept various expansion cards (not shown). In the implementation, low-speed controller 512 is coupled to storage device 508 and low-speed expansion port 514. The low-speed expansion port, which can include various communication ports, e.g., USB, Bluetooth, Ethernet, wireless Ethernet can be coupled to one or more input/output devices, such as a keyboard, a pointing device, microphone/speaker pair, a scanner, or a networking device such as a switch or router, e.g., through a network adapter. The computing device 500 can be implemented in a number of different forms, as shown in the figure. For example, it can be implemented as a standard server 520, or multiple times in a group of such servers. It can also be implemented as part of a rack server system 524. In addition, it can be implemented in a personal computer such as a laptop computer 522. Alternatively, components from computing device 500 can be combined with other components in a mobile device (not shown), such as device 550. Each of such devices can contain one or more of computing device 500, 550, and an entire system can be made up of multiple computing devices 500, 550 communicating with each other.

The computing device 500 can be implemented in a number of different forms, as shown in the figure. For example, it can be implemented as a standard server 520, or multiple times in a group of such servers. It can also be implemented as part of a rack server system 524. In addition, it can be implemented in a personal computer such as a laptop computer 522. Alternatively, components from computing device 500 can be combined with other components in a mobile device (not shown), such as device 550. Each of such devices can contain one or more of computing device 500, 550, and an entire system can be made up of multiple computing devices 500, 550 communicating with each other.

Computing device 550 includes a processor 552, memory 564, and an input/output device such as a display 554, a communication interface 566, and a transceiver 568, among other components. The device 550 can also be provided with a storage device, such as a microdrive or other device, to provide additional storage. Each of the components 550, 552, 564, 554, 566, and 568, are interconnected using various buses, and several of the components can be mounted on a common motherboard or in other manners as appropriate.

The processor 552 can execute instructions within the computing device 550, including instructions stored in the memory 564. The processor can be implemented as a chipset of chips that include separate and multiple analog and digital processors. Additionally, the processor can be implemented using any of a number of architectures. For example, the processor 510 can be a CISC (Complex Instruction Set Computers) processor, a RISC (Reduced Instruction Set Computer) processor, or a MISC (Minimal Instruction Set Computer) processor. The processor can provide, for example, for coordination of the other components of the device 550, such as control of user interfaces, applications run by device 550, and wireless communication by device 550.

Processor 552 can communicate with a user through control interface 558 and display interface 556 coupled to a display 554. The display 554 can be, for example, a TFT (Thin-Film-Transistor Liquid Crystal Display) display or an OLED (Organic Light Emitting Diode) display, or other appropriate display technology. The display interface 556 can comprise appropriate circuitry for driving the display 554 to present graphical and other information to a user. The control interface 558 can receive commands from a user and convert them for submission to the processor 552. In addition, an external interface 562 can be provided in communication with processor 552, so as to enable near area communication of device 550 with other devices. External interface 562 can provide, for example, for wired communication in some implementations, or for wireless communication in other implementations, and multiple interfaces can also be used.

The memory 564 stores information within the computing device 550. The memory 564 can be implemented as one or more of a computer-readable medium or media, a volatile memory unit or units, or a non-volatile memory unit or units. Expansion memory 574 can also be provided and connected to device 550 through expansion interface 572, which can include, for example, a SIMM (Single In Line Memory Module) card interface. Such expansion memory 574 can provide extra storage space for device 550, or can also store applications or other information for device 550. Specifically, expansion memory 574 can include instructions to carry out or supplement the processes described above, and can also include secure information. Thus, for example, expansion memory 574 can be provided as a security module for device 550, and can be programmed with instructions that permit secure use of device 550. In addition, secure applications can be provided via the SIMM cards, along with additional information, such as placing identifying information on the SIMM card in a non-hackable manner.

The memory can include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 564, expansion memory 574, or memory on processor 552 that can be received, for example, over transceiver 568 or external interface 562.

Device 550 can communicate wirelessly through communication interface 566, which can include digital signal processing circuitry where necessary. Communication interface 566 can provide for communications under various modes or protocols, such as GSM voice calls, SMS, EMS, or MMS messaging, CDMA, TDMA, PDC, WCDMA, CDMA2000, or GPRS, among others. Such communication can occur, for example, through radio-frequency transceiver 568. In addition, short-range communication can occur, such as using a Bluetooth, Wi-Fi, or other such transceiver (not shown). In addition, GPS (Global Positioning System) receiver module 570 can provide additional navigation- and location-related wireless data to device 550, which can be used as appropriate by applications running on device 550.

Device 550 can also communicate audibly using audio codec 560, which can receive spoken information from a user and convert it to usable digital information. Audio codec 560 can likewise generate audible sound for a user, such as through a speaker, e.g., in a handset of device 550. Such sound can include sound from voice telephone calls, can include recorded sound, e.g., voice messages, music files, etc. and can also include sound generated by applications operating on device 550.

The computing device 550 can be implemented in a number of different forms, as shown in the figure. For example, it can be implemented as a cellular telephone 580. It can also be implemented as part of a smartphone 582, personal digital assistant, or other similar mobile device.

Various implementations of the systems and methods described here can be realized in digital electronic circuitry, integrated circuitry, specially designed ASICs (application specific integrated circuits), computer hardware, firmware, software, and/or combinations of such implementations. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device, e.g., magnetic discs, optical disks, memory, Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device, e.g., a CRT (cathode ray tube) or LCD (liquid crystal display) monitor for displaying information to the user and a keyboard and a pointing device, e.g., a mouse or a trackball by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input.

The systems and techniques described here can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the systems and techniques described here, or any combination of such back end, middleware, or front end components. The components of the system can be interconnected by any form or medium of digital data communication, e.g., a communication network. Examples of communication networks include a local area network ("LAN"), a wide area network ("WAN"), and the Internet.

The computing system can include clients and servers. A client and server are generally remote from each other and typically interact through a communication network. The relationship of client and server arises by virtue of computer programs running on the respective computers and having a client-server relationship to each other.

### OTHER EMBODIMENTS

A number of embodiments have been described. Nevertheless, it will be understood that various modifications can be made without departing from the spirit and scope of the invention. In addition, the logic flows depicted in the figures do not require the particular order shown, or sequential order, to achieve desirable results. In addition, other steps can be provided, or steps can be eliminated, from the described flows, and other components can be added to, or removed from, the described systems. Accordingly, other embodiments are within the scope of the following claims.

### EXPERIMENTAL RESULTS

### Statistical and numerical examples of the compression method according to the invention

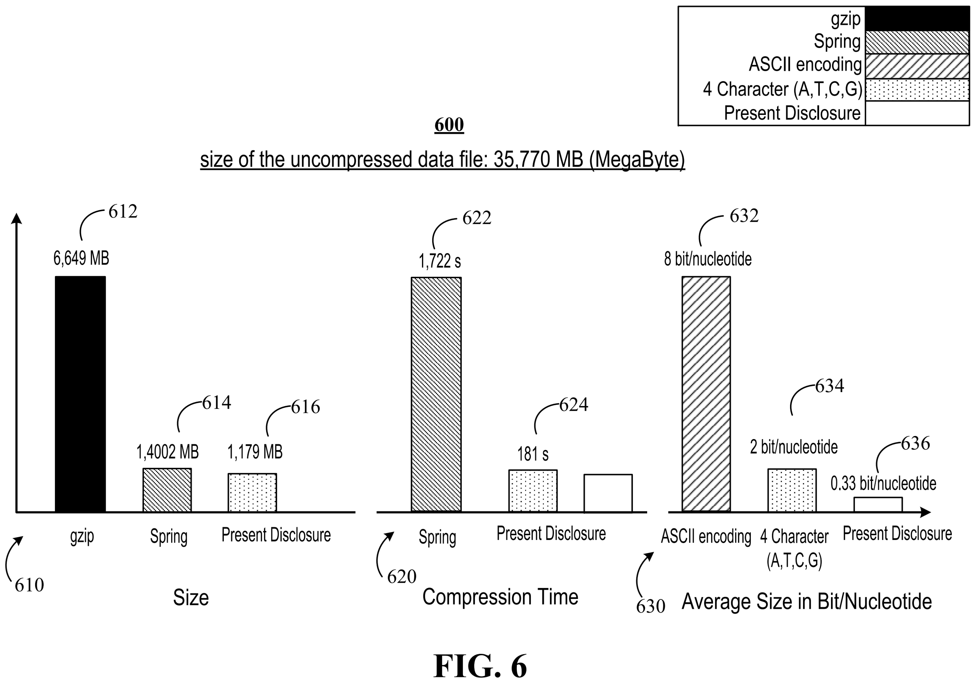

The following comparative example has been performed on an uncompressed data file of size 35,770 MB that contained 48 million reads or sequences of nucleotides. The results of this comparative example are also graphically depicted in figure 6.

The following results indicate a size of a compressed version of the uncompressed file of size 35,770 MB with 48 million reads when compressed using each respective algorithm. These results are depicted in chart 610.
o size of the file that has been compressed with the gzip software: 6,649 MB (612)
∘ size of the file that has been compressed with the non-reference-based SPRING software: 1,402 MB (614)
∘ size of the file that has been compressed with the reference-based compression method according to the present disclosure: 1,179 MB (6160

The following results indicate the amount of time it took to compare the uncompressed file of size 35,770 MB with 48 million reads using each respective algorithm. These results are depicted in chart 620.
∘ compression time with the non-reference-based SPRING software: 1,722 s (622)
∘ compression time with the reference based-compression method according to the present invention: 181 s (624)

The following result results indicate an average size in bit / nucleotide ration of the compressed version of the uncompressed file of size 35,770MB with 48 million reads when compressed using each respective algorithm. These results are depicted in chart 630.
∘ average size in Bit/Nucleotide of the uncompressed data file (ASCII encoding): 8 bit/nucleotide (630)
∘ average size in Bit/Nucleotide of the file that has been compressed with a coding adapted to 4 possible characters A, T, C, G: 2 bit/nucleotide (634)
∘ average size in Bit/Nucleotide of the file that has been compressed with the reference-based compression method according to the present invention: 0.33 bit/nucleotide (636)

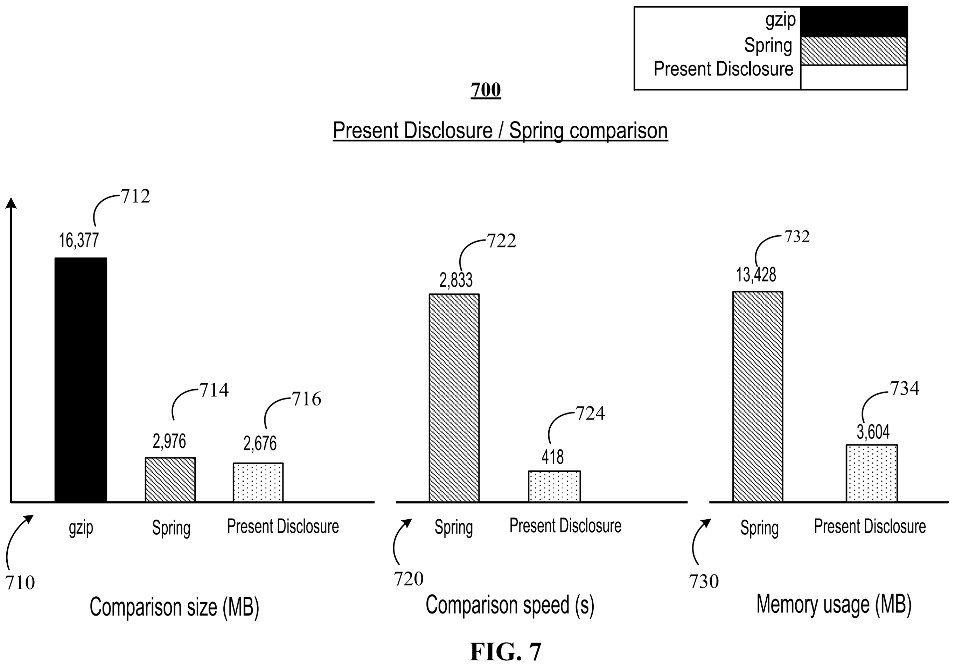

Figure 7 depicts additional comparative results using different compression algorithms to compress sample reads generated using WXS novaseq and a reference genome SRR8604734.

The chart 710 shows a comparison in resulting compression size (MB) between gzip's compression of WXS novaseq reads using reference genome SRR8604734 (712), Spring's compression of WXS novaseq reads using reference genome SRR8604734 (716), and the present disclosure's compression of compression of WXS novaseq reads using reference genome SRR8604734 (716). Measurements are in megabytes.

The chart 720 shows a comparison of compression speeds of the compression algorithms in 720 to compress WXS novaseq reads using reference genome SRR8604734. Spring compression speeds to compress WXS novaseq reads using reference genome SRR8604734 are shown in (722) in comparison with the speed of the present disclosure to compress WXS novaseq reads using reference genome SRR8604734 shown in 724. Measurements are in seconds.

The chart 730 shows a comparison of the memory usage of the compression algorithms in 730 during compression of WXS novaseq reads using reference genome SRR8604734. Spring compression utilized 13,428 MB of memory to compress WXS novaseq reads using reference genome SRR8604734 732 and the present disclosure used 3,604 MB of memory to compress WXS novaseq reads using reference genome SRR8604734 (734). Measurements are in megabytes.

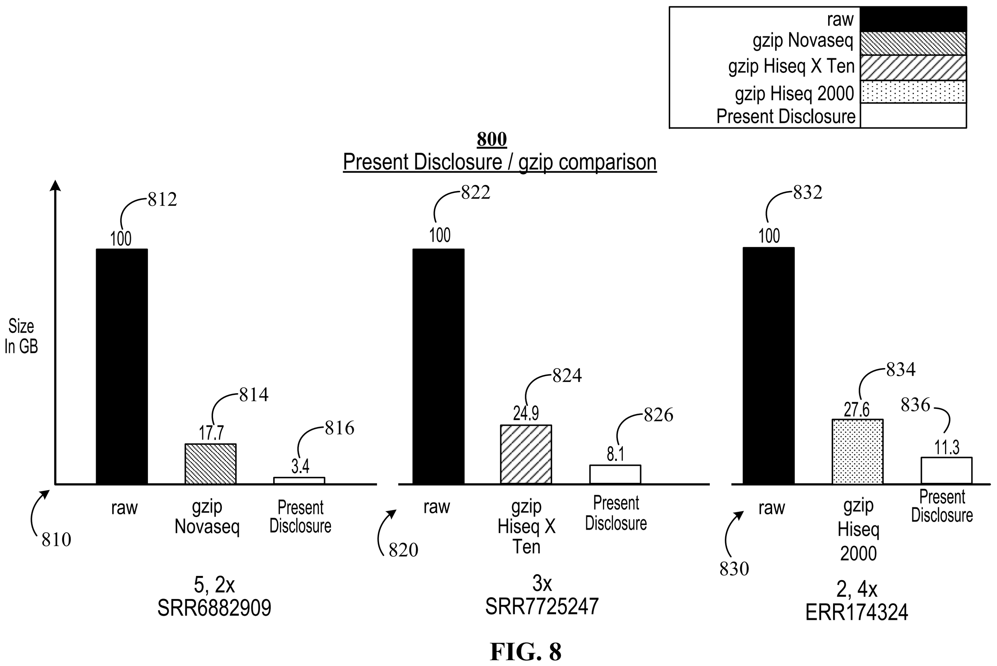

Figure 8 depicts additional comparative results using different compression algorithms to compress sample reads generated using different sequeners and different reference genomes at different compression rations.

The chart 810 shows the raw size (812) in gigabytes (GB) of a file of reads generated using Novaseq. Using gzip to compress the Novaseq reads of size 100 GB, gzip compressed the raw data into 17.7 GB (814). The present disclosure compressed the same raw size 812 file of 100 GB Novaseq generated reads into a compressed file 3.4 GB (816). The reference genome used for the compression by both gzip and the present disclosure, as shown in 810, was SRR6882909 and compression ratio was 5.2x.

The chart 820 shows the raw size (822) in gigabytes (GB) of a file of reads generated using Hiseq X Ten. Using gzip to compress Hiseq X Ten reads of size 100 GB, gzip compressed the raw data into 24.9 GB (824). The present disclosure compressed the same raw size 822 file of 100 GB Hiseq X Ten generated reads into a compressed file of 8.1 GB (826). The reference genome used for the compression by both gzip and the present disclosure, as shown in 820, was SRR7725247 and compression ratio was 3x.

The chart 830 shows the raw size (832) in gigabytes (GB) of a file of reads generated using Hiseq 2000. Using gzip to compress Hiseq 2000 reads of size 100 GB, gzip compressed the raw data into 27.6 GB (834). The present disclosure compressed the same raw size 832 file of 100 GB Hiseq 2000 generated reads into a compressed file of 11.3 GB (836). The reference genome used for the compression by both gzip and the present disclosure, as shown in 830, was ERR174324 and compression ratio was 2.4x.

The numerical examples indicated above illustrate that the present invention allows for fast compression and decompression, while providing a high compression ratio.

Aspects can be understood from the following numbered paragraphs:
1. A computer-implemented method for the compression of genome sequence data produced by a sequencing machine, said genome sequence data comprising reads of sequences of nucleotides or bases that have been aligned to a reference sequence, thereby creating aligned reads, said aligned reads being stored as a list of reads in an initial file, said method comprising:
   for each aligned read, determining whether said read is perfectly or imperfectly mapped with said reference sequence or whether said read is unmapped with said reference sequence,
   encoding the reads according to said determination, wherein the reads that are determined to be perfectly mapped are encoded according to a first encoding process and the reads that are determined to be unmapped are encoded according to a second encoding process,
   wherein the determining step comprises comparing, for each imperfectly mapped read, the number of mismatches between said read and said reference sequence with a threshold value,
   wherein, in the encoding step, the reads that are determined to be imperfectly mapped are encoded according to the second encoding process or to a third encoding process, the imperfectly mapped reads being encoded according to the second encoding process when said number of mismatches is greater than the threshold value, and the imperfectly mapped reads being encoded according to the third encoding process when said number of mismatches is lower than the threshold value,
   wherein, in said second encoding process, each nucleotide or base of the read is individually encoded,
   wherein said first and third encoding processes comprise distinct sets of descriptors, each set of descriptors univocally representing the reads associated to a corresponding encoding process, each of said first and third encoding processes being a reduced information source entropy encoding process.
2. The method of paragraph 1, wherein the determining step comprises, when a read is determined to be imperfectly mapped with the reference sequence and has a number of mismatches lower than the threshold value, a further determination as to whether the read is globally or locally mapped with said reference sequence, and wherein the third encoding process comprises a first encoding subprocess and a second encoding subprocess, the reads that are determined to be globally mapped being encoded according to the first encoding subprocess, the reads that are determined to be locally mapped being encoded according to the second encoding subprocess, said first and second encoding subprocesses comprising distinct sets of descriptors, each set of descriptors univocally representing the reads associated to a corresponding encoding subprocess.
3. The method of paragraph 2, wherein said descriptors of said first encoding subprocess comprise an alignment start position in the reference sequence, a read length and a list of mismatches by substitutions of symbols, and wherein said descriptors of said second encoding subprocess comprise a local alignment start position in the reference sequence, a read length, a list of mismatches by substitutions of symbols, and a length of the clipped portions of the read that are not part of the alignment.
4. The method of paragraph 3, wherein, in the encoding step, the clipped portions of a read that is to be encoded according to the second encoding subprocess are concatenated, each nucleotide or base of said clipped portions being individually encoded.
5. The method of any of the preceding paragraphs, wherein, in the encoding step, each mismatch of an imperfectly mapped read is encoded on 1 byte.
6. The method of paragraph 5, wherein, in the encoding step, each mismatch of an imperfectly mapped read is encoded as follows:
   - two first bits of the byte are used to encode an alternate nucleotide or base present in the read instead of a corresponding reference nucleotide or base in the reference sequence; and
   - six last bits of the byte are used to encode a position of the mismatch in the reference sequence, said position being computed as an offset from a previous mismatch of the read.
7. The method of paragraph 6, wherein, in the encoding step, if the offset computed between a given mismatch and the previous mismatch is greater than a maximum encodable value, then at least one fake mismatch is inserted between said two mismatches until every offset between each of said mismatches and said at least one fake mismatch is lower than said maximum encodable value, a fake mismatch being defined as a mismatch for which bits of the byte used to encode the mismatch or to encode a nucleotide or base that is equal to the corresponding reference nucleotide or base in the reference sequence.
8. The method of any of the preceding paragraphs, further comprising an initial step of dividing the list of reads into blocks of reads, with each block beginning with a header containing information needed to decode the block, wherein said compression method is performed block by block.
9. The method of paragraph 8, wherein the blocks of reads have the same block size.
10. The method of any of the preceding paragraphs, further comprising a final step of providing a compressed file comprising a list of encoded reads, said encoded reads being stored in the compressed file in the same order as that of the reads stored in the initial file.
11. The method of any of the preceding paragraphs, wherein said threshold value is equal to 31.
12. The method of any of the preceding paragraphs, further comprising, for each aligned read, a step of determining whether said read comprises at least one mismatch corresponding to a case in which the sequencing machine was unable to call any base or nucleotide.
13. The method of paragraph 12, further comprising, for each read comprising at least one mismatch corresponding to a case in which the sequencing machine was unable to call any base or nucleotide, a step of determining the number of such mismatches and a step of comparing said number with a reference threshold value.
14. The method of paragraph 13, wherein, in the encoding step, if the number of such mismatches is greater than the reference threshold value, each nucleotide or base of a read that is to be encoded according to the second encoding process is individually encoded on 4 bits, and, if the number of such mismatches is lower than the reference threshold value, each nucleotide or base of a read that is to be encoded according to the second encoding process is individually encoded on 2 bits and the encoding step further comprises encoding a list of positions along the reference sequence, said positions corresponding to the positions of such mismatches in the reference sequence.
15. A computer program product embodied on a computer readable storage medium, said computer program product comprising computer executable instructions which, when executed by a processor, cause the processor to perform operations comprising the steps of the method of any of the preceding paragraphs.
16. A computer readable storage medium having computer executable instructions which, when executed by a processor, cause the processor to perform operations comprising the steps of the method of any of paragraphs 1-14.
17. An apparatus, comprising:
   a processor; and
   a memory operatively coupled to the processor to form a computing device, the memory storing processor-executable instructions which, based at least on being executed on the processor, cause the processor to perform operations comprising the steps of the method of paragraph 1.
18. A method for compressing genomic sequence data, the method comprising:
   accessing, by the one or more processors, a storage device storing a plurality of read records in manner that preserves a sequence ordering of the read records as the produced by a mapping and aligning module;
   for each particular read record of the plurality of read records:
      obtaining, by the one or more processors, the particular read record;
      determining, by the one or more processors, whether the particular read record corresponds to a read that is perfectly mapped to a reference sequence or imperfectly mapped to the reference sequence;
      based on determining, by the one or more processors, that the particular read record corresponds to a read that is imperfectly mapped to the reference sequence, determining, by the one or more processors, whether a number of mismatches of the imperfectly mapped read satisfies a predetermined threshold number of mismatches;
      based on determining that the number of mismatches satisfies the predetermined threshold number of mismatches, encoding, by the one or more processors, each mismatch of the imperfectly mapped read into a compressed record having a predetermined compressed record size; and
      storing, by the one or more processors, the compressed record in the storage device while maintaining the sequence ordering of the read records.
19. The method of paragraph 18, wherein each read record of the plurality of read records comprises:
   data indicating an absolute starting position of the aligned read with respect to the reference sequence,
   data indicating a length of the read,
   data indicating whether the read is perfectly mapped or imperfectly mapped,
   data indicating a number of mismatches identified in the read,
   data indicating whether the read includes at least one undetermined base N,
   data indicating a number of undetermined bases N in the read,
   data indicating whether the read is mapped or unmapped,
   data indicating a position of the read record in a sequence of read records output by the mapping and aligning module, and
   data indicating a relative position of said possible mismatches in the read.
20. The method of paragraph 18, wherein the predetermined compressed record size is one byte.
21. The method of paragraph 20, wherein encoding each mismatch of the imperfectly mapped read into a compressed record having a size of one byte comprises for each particular mismatch:
   encoding, by one or more processors, a first two bits of the byte to include data representing an alternate nucleotide or base present in the read instead of a corresponding reference nucleotide or base in the reference sequence; and
   encoding, by one or more processors, a six remaining bits of the byte to include data representing a position of the mismatch in the reference sequence, said position being computed as an offset from a previous mismatch of the read.
22. The method of paragraph 21, the method further comprising:
   determining, by one or more processors, whether the offset is greater than a maximum encodable value;
   based on determining that the offset is greater than the maximum encoded value, inserting, by one or more processors, at least one fake mismatch between the particular mismatch and the previous mismatch.
23. The method of paragraph 18, wherein the method further comprises:
   based on determining that the number of mismatches does not satisfy the predetermined threshold number of mismatches, encoding, by one or more processors, a list of positions of the reference sequence corresponding to a position of each of the mismatches to the reference sequence using a reduced information entropy encoding process.
24. The method of paragraph 18, wherein the method further comprises:
   based on determining that the read record corresponds to a read that is perfectly mapped to the reference sequence, encoding, by the one or more processors, at least a portion of the read record using reduced information entropy encoding.
25. The method of paragraph 18, wherein determining, by the one or more computers, whether a number of mismatches of the imperfectly mapped read satisfies a predetermined threshold number of mismatches comprises:
   determining, by the one or more processors, whether the number of mismatches of the imperfectly mapped read is greater than the reference threshold.
26. A system for compressing genomic sequence data, the system comprising:
   one or more computers and one or more storage devices storing instructions that are operable, when executed by one or more computers, to cause the one or more computers to perform the operations comprising:
   accessing, by the one or more computers, a storage device storing a plurality of read records in manner that preserves a sequence ordering of the read records as the produced by a mapping and aligning module;
   for each particular read record of the plurality of read records:
      obtaining, by the one or more computers, the particular read record;
      determining, by the one or more computers, whether the particular read record corresponds to a read that is perfectly mapped to a reference sequence or imperfectly mapped to the reference sequence;
      based on determining, by the one or more computers, that the particular read record corresponds to a read that is imperfectly mapped to the reference sequence, determining, by the one or more computers, whether a number of mismatches of the imperfectly mapped read satisfies a predetermined threshold number of mismatches;
      based on determining that the number of mismatches satisfies the predetermined threshold number of mismatches, encoding, by the one or more computers, each mismatch of the imperfectly mapped read into a compressed record having a predetermined compressed record size; and
      storing, by the one or more computers, the compressed record in the storage device while maintaining the sequence ordering of the read records.
27. The system of paragraph 26, wherein each read record of the plurality of read records comprises:
   data indicating an absolute starting position of the aligned read with respect to the reference sequence,
   data indicating a length of the read,
   data indicating whether the read is perfectly mapped or imperfectly mapped,
   data indicating a number of mismatches identified in the read,
   data indicating whether the read includes at least one undetermined base N,
   data indicating a number of undetermined bases N in the read,
   data indicating whether the read is mapped or unmapped,
   data indicating a position of the read record in a sequence of read records output by the mapping and aligning module, and
   data indicating a relative position of said possible mismatches in the read.
28. The system of paragraph 26, wherein the predetermined compressed record size is one byte.
29. The system of paragraph 28, wherein encoding each mismatch of the imperfectly mapped read into a compressed record having a size of one byte comprises for each particular mismatch:
   encoding, by one or more computers, a first two bits of the byte to include data representing an alternate nucleotide or base present in the read instead of a corresponding reference nucleotide or base in the reference sequence; and
   encoding, by one or more computers, a six remaining bits of the byte to include data representing a position of the mismatch in the reference sequence, said position being computed as an offset from a previous mismatch of the read.
30. The system of paragraph 29, the operations further comprising:
   determining, by the one or more computers, whether the offset is greater than a maximum encodable value;
   based on determining that the offset is greater than the maximum encoded value, inserting, by the one or more computers, at least one fake mismatch between the particular mismatch and the previous mismatch.
31. The system of paragraph 26, the operations further comprising:
   based on determining that the number of mismatches does not satisfy the predetermined threshold number of mismatches, encoding, by one or more computers, a list of positions of the reference sequence corresponding to a position of each of the mismatches to the reference sequence using a reduced information entropy encoding process.
32. The system of paragraph 26, the operations further comprising:
   based on determining that the read record corresponds to a read that is perfectly mapped to the reference sequence, encoding, by one or more computers, at least a portion of the read record using reduced information entropy encoding.
33. The system of paragraph 26, wherein determining, by the one or more computers, whether a number of mismatches of the imperfectly mapped read satisfies a predetermined threshold number of mismatches comprises:
   determining, by the one or more computers, whether the number of mismatches of the imperfectly mapped read is greater than the predetermined threshold number of mismatches.
34. A computer-readable storage device having stored thereon instructions, which, when executed by a data processing apparatus, cause the data processing apparatus to perform operations for compressing genomic sequence data, the operations comprising:
   accessing a storage device storing a plurality of read records in manner that preserves a sequence ordering of the read records as the produced by a mapping and aligning module;
   for each particular read record of the plurality of read records:
      obtaining the particular read record;
      determining whether the particular read record corresponds to a read that is perfectly mapped to a reference sequence or imperfectly mapped to the reference sequence;
      based on determining that the particular read record corresponds to a read that is imperfectly mapped to the reference sequence, determining whether a number of mismatches of the imperfectly mapped read satisfies a predetermined threshold number of mismatches;
      based on determining that the number of mismatches satisfies the predetermined threshold number of mismatches, encoding each mismatch of the imperfectly mapped read into a compressed record having a predetermined compressed record size; and
      storing the compressed record in the storage device while maintaining the sequence ordering of the read records.
35. The computer-readable storage device of paragraph 34, wherein each read record of the plurality of read records comprises:
   data indicating an absolute starting position of the aligned read with respect to the reference sequence,
   data indicating a length of the read,
   data indicating whether the read is perfectly mapped or imperfectly mapped,
   data indicating a number of mismatches identified in the read,
   data indicating whether the read includes at least one undetermined base N,
   data indicating a number of undetermined bases N in the read,
   data indicating whether the read is mapped or unmapped,
   data indicating a position of the read record in a sequence of read records output by the mapping and aligning module, and
   data indicating a relative position of said possible mismatches in the read.
36. The computer-readable storage device of paragraph 34, wherein the predetermined compressed record size is one byte.
37. The computer-readable storage device of paragraph 36, wherein encoding each mismatch of the imperfectly mapped read into a compressed record having a size of one byte comprises for each particular mismatch:
   encoding a first two bits of the byte to include data representing an alternate nucleotide or base present in the read instead of a corresponding reference nucleotide or base in the reference sequence; and
   encoding a six remaining bits of the byte to include data representing a position of the mismatch in the reference sequence, said position being computed as an offset from a previous mismatch of the read.
38. The computer-readable storage device of paragraph 37, the operations further comprising:
   determining whether the offset is greater than a maximum encodable value;
   based on determining that the offset is greater than the maximum encoded value, inserting at least one fake mismatch between the particular mismatch and the previous mismatch.
39. The computer-readable storage device of paragraph 37, the operations further comprising:
   based on determining that the number of mismatches does not satisfy the predetermined threshold number of mismatches, encoding a list of positions of the reference sequence corresponding to a position of each of the mismatches to the reference sequence using a reduced information entropy encoding process.
40. The computer-readable storage device of paragraph 37, the operations further comprising:
   based on determining that the read record corresponds to a read that is perfectly mapped to the reference sequence, encoding at least a portion of the read record using reduced information entropy encoding.
41. The computer-readable storage device of paragraph 37, wherein determining whether a number of mismatches of the imperfectly mapped read satisfies a predetermined threshold number of mismatches comprises:
   determining whether the number of mismatches of the imperfectly mapped read is greater than the predetermined threshold number of mismatches.
42. A hardware processor that includes hardware processing circuitry that is configured to perform one or more operations, the one or more operations comprising:
   accessing, by the hardware processing circuitry, a storage device storing a plurality of read records in manner that preserves a sequence ordering of the read records as the produced by a mapping and aligning module;
   for each particular read record of the plurality of read records:
      obtaining, by the hardware processing circuitry, the particular read record;
      determining, by the hardware processing circuitry, whether the particular read record corresponds to a read that is perfectly mapped to a reference sequence or imperfectly mapped to the reference sequence;
      based on determining, by the hardware processing circuitry, that the particular read record corresponds to a read that is imperfectly mapped to the reference sequence, determining, by the hardware processing circuitry, whether a number of mismatches of the imperfectly mapped read satisfies a predetermined threshold number of mismatches;
      based on determining that the number of mismatches satisfies the predetermined threshold number of mismatches, encoding, by the hardware processing circuitry, each mismatch of the imperfectly mapped read into a compressed record having a predetermined compressed record size; and
      storing, by the hardware processing circuitry, the compressed record in the storage device while maintaining the sequence ordering of the read records.
43. The hardware processor of paragraph 42, wherein each read record of the plurality of read records comprises:
   data indicating an absolute starting position of the aligned read with respect to the reference sequence,
   data indicating a length of the read,
   data indicating whether the read is perfectly mapped or imperfectly mapped,
   data indicating a number of mismatches identified in the read,
   data indicating whether the read includes at least one undetermined base N,
   data indicating a number of undetermined bases N in the read,
   data indicating whether the read is mapped or unmapped,
   data indicating a position of the read record in a sequence of read records output by the mapping and aligning module, and
   data indicating a relative position of said possible mismatches in the read.
44. The hardware processor of paragraph 42, wherein the predetermined compressed record size is one byte.
45. The hardware processor of paragraph 44, wherein encoding each mismatch of the imperfectly mapped read into a compressed record having a size of one byte comprises for each particular mismatch:
   encoding, by the hardware processing circuitry, a first two bits of the byte to include data representing an alternate nucleotide or base present in the read instead of a corresponding reference nucleotide or base in the reference sequence; and
   encoding, by the hardware processing circuitry, a six remaining bits of the byte to include data representing a position of the mismatch in the reference sequence, said position being computed as an offset from a previous mismatch of the read.
46. The hardware processor of paragraph 45, the hardware processor further comprising:
   determining, by the hardware processing circuitry, whether the offset is greater than a maximum encodable value;
   based on determining that the offset is greater than the maximum encoded value, inserting, by the hardware processing circuitry, at least one fake mismatch between the particular mismatch and the previous mismatch.
47. The hardware processor of paragraph 45, the hardware processor further comprising:
   based on determining that the number of mismatches does not satisfy the predetermined threshold number of mismatches, encoding, by the hardware processing circuitry, a list of positions of the reference sequence corresponding to a position of each of the mismatches to the reference sequence using a reduced information entropy encoding process.
48. The hardware processor of paragraph 45, the hardware processor further comprising:
   based on determining that the read record corresponds to a read that is perfectly mapped to the reference sequence, encoding, by the hardware processing circuitry, at least a portion of the read record using reduced information entropy encoding.
49. The hardware processor of paragraph 45, wherein determining, by the hardware processing circuitry, whether a number of mismatches of the imperfectly mapped read satisfies a predetermined threshold number of mismatches comprises:
   determining, by the hardware processing circuitry, whether the number of mismatches of the imperfectly mapped read is greater than the predetermined threshold number of mismatches.

## Claims

1. A computer-implemented method for the compression of genome sequence data produced by a sequencing machine, said genome sequence data comprising reads of sequences of nucleotides or bases that have been aligned to a reference sequence, thereby creating aligned reads, said aligned reads being stored as a list of reads in an initial file, said method comprising:
for each aligned read, determining whether said read is perfectly mapped with said reference sequence, imperfectly mapped with said reference sequence, or unmapped with said reference sequence; and
encoding the reads according to said determination,
wherein the method comprises comparing, for each imperfectly mapped read, the number of mismatches between said read and said reference sequence with a threshold value, and
wherein the encoding step comprises, for one or more imperfectly mapped reads for which said number of mismatches is lower than the threshold value,
encoding at least one mismatch of the read on 1 byte, wherein two first bits of the byte are used to encode an alternate nucleotide or base present in the read instead of a corresponding reference nucleotide or base in the reference sequence, and six last bits of the byte are used to encode a position of the mismatch, said position being computed as an offset from a previous mismatch of the read.

2. The method of claim 1, wherein the reads that are determined to be perfectly mapped are encoded according to a first encoding process or a second encoding process and the reads that are determined to be unmapped are encoded according to the second encoding process,
wherein the reads that are determined to be imperfectly mapped are encoded according to the second encoding process or a third encoding process, the imperfectly mapped reads being encoded according to the second encoding process when said number of mismatches is greater than the threshold value,
wherein, in said second encoding process, each nucleotide or base of the read is individually encoded,
wherein said first and third encoding processes comprise distinct sets of descriptors, each set of descriptors univocally representing the reads associated to a corresponding encoding process, each of said first and third encoding processes being a reduced information source entropy encoding process, and
wherein said third encoding process comprises said encoding at least one mismatch of the read on 1 byte, wherein two first bits of the byte are used to encode an alternate nucleotide or base present in the read instead of a corresponding reference nucleotide or base in the reference sequence, and six last bits of the byte are used to encode a position of the mismatch, said position being computed as an offset from a previous mismatch of the read.

3. The method of claim 2, wherein the method comprises, when a read is determined to be imperfectly mapped with the reference sequence and has a number of mismatches lower than the threshold value, a further determination as to whether the read is globally or locally mapped with said reference sequence, and wherein the third encoding process comprises a first encoding subprocess and a second encoding subprocess, the reads that are encoded according to the third encoding process and determined to be globally mapped being encoded according to the first encoding subprocess, the reads that are encoded according to the third encoding process and determined to be locally mapped being encoded according to the second encoding subprocess, said first and second encoding subprocesses comprising distinct sets of descriptors, each set of descriptors univocally representing the reads associated to a corresponding encoding subprocess.

4. The method of claim 3, wherein said descriptors of said first encoding subprocess comprise an alignment start position in the reference sequence, a read length and a list of mismatches by substitutions of symbols, and wherein said descriptors of said second encoding subprocess comprise a local alignment start position in the reference sequence, a read length, a list of mismatches by substitutions of symbols, and a length of the clipped portions of the read that are not part of the alignment, optionally wherein, in the encoding step, the clipped portions of a read that is to be encoded according to the second encoding subprocess are concatenated, each nucleotide or base of said clipped portions being individually encoded.

5. The method of claim 1, wherein, in the encoding step, if the offset computed between a given mismatch and the previous mismatch is greater than a maximum encodable value, then at least one fake mismatch is inserted between said two mismatches until every offset between each of said mismatches and said at least one fake mismatch is lower than said maximum encodable value, a fake mismatch being defined as a mismatch for which bits of the byte used to encode the mismatch encode a nucleotide or base that is equal to the corresponding reference nucleotide or base in the reference sequence.

6. The method of any one of the preceding claims, further comprising an initial step of dividing the list of reads into blocks of reads, with each block beginning with a header containing information needed to decode the block, wherein said compression method is performed block by block, optionally wherein the blocks of reads have the same block size.

7. The method of any one of the preceding claims, further comprising a final step of providing a compressed file comprising a list of encoded reads, said encoded reads being stored in the compressed file in the same order as that of the reads stored in the initial file.

8. The method of any one of the preceding claims, wherein said threshold value is equal to 31.

9. The method of any one of claims 2 to 8, further comprising, for each aligned read, a step of determining whether said read comprises at least one mismatch corresponding to a case in which the sequencing machine was unable to call any base or nucleotide.

10. The method of claim 9, further comprising, for each read comprising at least one mismatch corresponding to a case in which the sequencing machine was unable to call any base or nucleotide, a step of determining the number of such mismatches and a step of comparing said number with a reference threshold value.

11. The method of claim 10, wherein, if a read is determined to be perfectly mapped, and if said read comprises no mismatches corresponding to a case in which the sequencing machine was unable to call any base or nucleotide or has a number of such mismatches lower than the reference threshold value, then said read is encoded according to the first encoding process, and if the read has a number of such mismatches greater than the reference threshold value, then said read is encoded according to the second encoding process.

12. The method of claim 10, wherein, in the encoding step, if the number of such mismatches is greater than the reference threshold value, each nucleotide or base of a read that is to be encoded according to the second encoding process is individually encoded on 4 bits, and, if the number of such mismatches is lower than the reference threshold value, each nucleotide or base of a read that is to be encoded according to the second encoding process is individually encoded on 2 bits and the encoding step further comprises encoding a list of positions along the reference sequence, said positions corresponding to the positions of such mismatches in the reference sequence.

13. The method of claim 10, wherein if a read is determined to be imperfectly mapped with a number of mismatches lower than the threshold value, and if said read comprises no mismatches corresponding to a case in which the sequencing machine was unable to call any base or nucleotide or has a number of such mismatches lower than the reference threshold value, then said read is encoded according to the third encoding process, and if the read has a number of such mismatches greater than the reference threshold value, then said read is encoded according to the second encoding process.

14. A computer readable storage medium having computer executable instructions which, when executed by a processor, cause the processor to perform operations comprising the steps of the method of any one of claims 1-13.

15. An apparatus, comprising:
a processor; and
a memory operatively coupled to the processor to form a computing device, the memory storing processor-executable instructions which, based at least on being executed on the processor, cause the processor to perform operations comprising the steps of the method of any one of claims 1-13.
